Europäisches Patentamt

(19) European Patent Office

Office européen des brevets

(11) Publication number : **0 484 017 A2**

# EUROPEAN PATENT APPLICATION

(21) Application number : 91309645.9

(22) Date of filing : 18.10.91

(51) Int. Cl.⁵ : **C12N 15/16,** C12P 21/02, C07K 13/00, A61K 37/24, C12N 15/12, // (A61K37/54, 37:24)

(30) Priority : 19.10.90 US 598877

(43) Date of publication of application : 06.05.92 Bulletin 92/19

(84) Designated Contracting States : AT BE CH DE DK ES FR GB GR IT LI LU NL SE

(71) Applicant : **THE GENERAL HOSPITAL CORPORATION**
**55 Fruit Street**
**Boston, MA 02114 (US)**

(72) Inventor : **Quertermous, Thomas**
**240 Brattle Street, Apt. 41**
**Cambridge, Massachusetts 02148 (US)**
Inventor : **Bloch, Kenneth**
**10 Fernald Drive, Apt. 34**
**Cambridge, Massachusetts 02138 (US)**

(74) Representative : **Sheard, Andrew Gregory et al**
**Kilburn & Strode 30, John Street**
**London WC1N 2DD (GB)**

(54) Endothelin precursors, genes and receptors.

(57)     The amino acid and nucleotide sequences of precursor molecules for endothelin-2 and endothelin-3 are disclosed. In addition, the purification and cloning of the receptors for ET-1, ET-2, and ET-3 are described. Compositions useful in regulating vascular activities are also disclosed.

EP 0 484 017 A2

The invention relates to compositions and methods for modulation of cardiovascular reactions.

The endothelin genes encode a family of potent vasoactive peptides. Endothelin-1 (ET-1), the first of these peptides to be characterized, was isolated from the supernatants of cultured porcine aortic endothelial cells. ET-1 is a 21 amino acid peptide. The peptide is a vasoconstrictor in vitro and increases blood pressure in vivo. In addition, ET-1 is a mitogen for smooth muscle cells, mesangial cells, and fibroblasts (Bloch et al., J. Biol. Chem. 264:10851 (1989)). The peptide further inhibits renin release from glomeruli and stimulates atrial natriuretic factor secretion from atrial cardiocytes.

The human ET-1 precusor, preproendothelin-1, is 212 amino acids long. A 15-amino acid ET-1-like sequence homologous to ET-1, sharing eight residues including four cysteines, resides in the carboxyl terminal portion of the precursor.

Other genomic fragments have been isolated that encode vasoconstrictor peptides related to ET-1: endothelin-2 (ET-2) and endothelin-3 (ET-3) (Inoue et al., Proc. Natl. Acad. Sci. USA 86:2863 (1989)). Thus, a family of endothelin peptides exists in the mammalian cardiovascular system which may provide a mechanism for regulating blood pressure and/or local blood distribution.

The vascular endothelium is a simple squamous endothelium which is the layer of the intima in direct contact with the blood. Endothelial cells play a major role in capillary transport, regulation of plasma lipids, and the control of hemostasis. The endothelium has also been implicated in playing a role in the relaxation and contraction of isolated arteries and veins caused by a variety of vasoactive substances.

As more is known about the endothelium and the vasoactive substances expressed in endothelial cells and other cell types, it becomes apparent that the regulation of blood flow and blood pressure is quite complicated. Therefore, research is needed in this area, not only to understand the mechanisms which regulate vasoactivities, but also to propose methods for the control and regulation of blood pressure and blood flow.

Thus a first aspect of the present invention relates to a peptide (or an amino acid sequence) which is capable of being cleaved (for example, cleavable) to form ET-2 and/or ET-3, or a variant thereof.

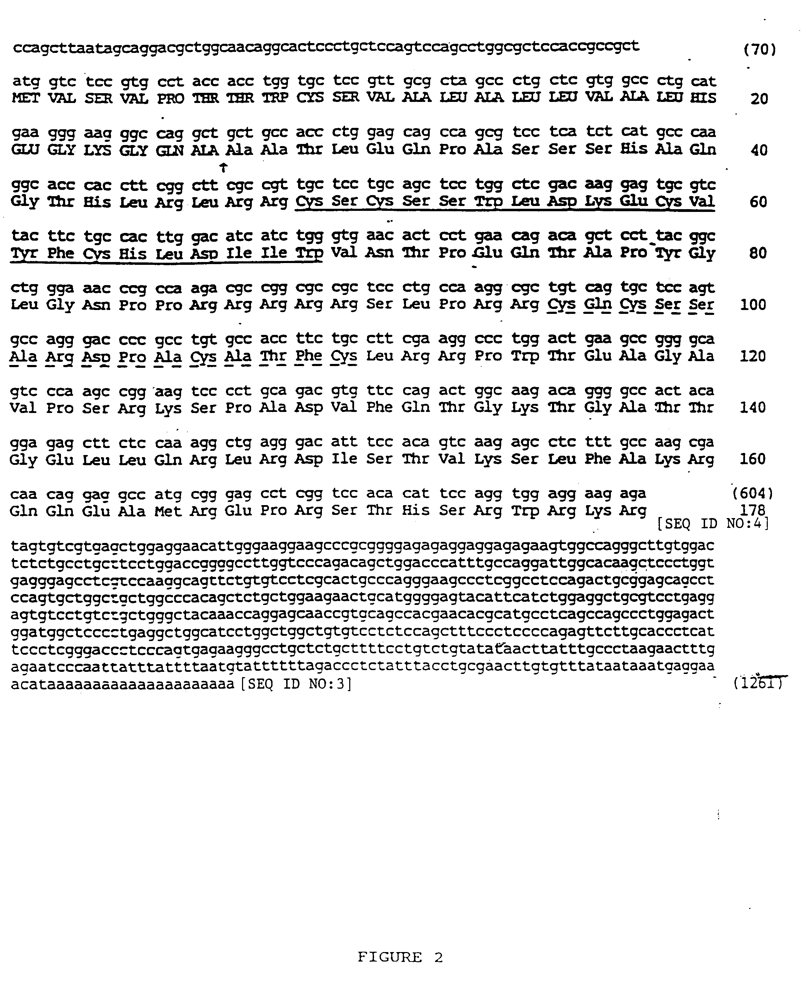

In one embodiment it is preferred that the peptide comprises the amino acid sequence of Figure 2 and/or is encoded by the nucleotide sequence given in Figure 2.

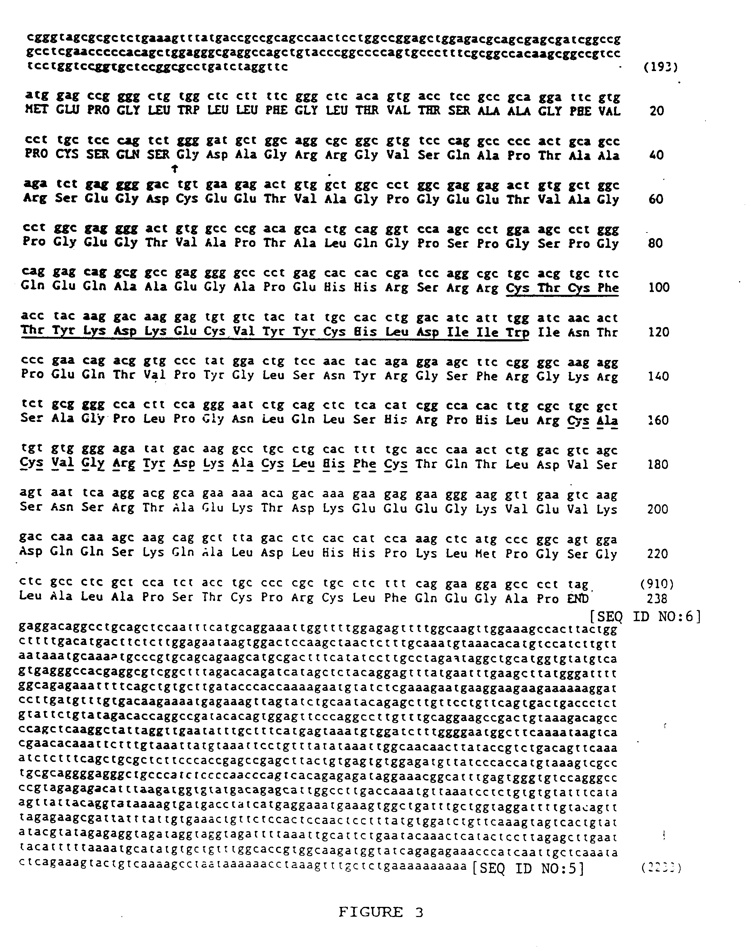

In other embodiments is is preferred that the peptide comprises the amino acid sequence of Figure 3 and/or is encoded by the nucleotide sequence given in Figure 3.

Here variants include biologically active fragments, analogues, chemical derivatives and in particular peptide that have been modified by deletion, addition or substitution of one or more amino acids. Such variants will usually have a similar biological activity, or substantially all of the biological activity, of the peptide of the present invention.

The peptides of the present invention may be advantageous in that they may possess a longer in vivo half-life than ET-2 or ET-3 (or a variant thereof) respectively.

If the peptide is capable of being cleaved to form ET-2 (or a variant thereof), then it is preferably equal to or at least 36, 59, 152 or 178 amino acids in length.

If the peptide is cleavable to form ET-3 (or a variant thereof) then the peptide is preferably equal to or greater than 44, 114, 213 or 238 amino acids in length.

However, it will be appreciated that the peptide of the present invention may be a peptide which is ± 5 amino acids in length from the lengths given in the previous two paragraphs.

A second aspect of the present invention relates to a peptide of the first aspect for use in medicine.

A third aspect of the present invention relates to a pharmaceutical composition comprising a peptide of the first aspect and a pharmaceutically acceptable carrier. The composition is suitably an injectable composition and in which case it will preferably be sterile. The composition may therefore be adapted for intravenous administration.

A fourth aspect relates to the use of a peptide of the first aspect in the preparation of:

(a) a blood pressure and/or vascular activity regulating or modulating agent;

(b) a vasoconstrictor and/or pressor agent;

(c) a regional vasodilatory agent;

(d) a vascular smooth muscle cell or fibroblast stimulating or proliferating agent;

(e) an airway and/or intestinal smooth muscle contracting agent;

(f) a myocardium positive inotropic and/or chronotropic agent;

(g) an iconsanoid or vascular bed endothelium-derived relaxing factor releasing agent;

(h) an atrial natriuretic peptide from atrial cardiocyte stimulating agent;

(i) a renin release from glomerulus inhibiting agent; and/or

(j) a norepinephrine release from sympathetic terminal modulating agent.

A fifth aspect of the present invention relates to a product comprising a peptide of the first aspect and a cleavage agent adapted to cleave a peptide to form ET-2 and/or ET-3 and/or a variant thereof, as a combined preparation for simultaneous, separate or sequential use in vascular, cardiovascular, blood pressure regulatory, vasoconstrictor, vasodilatory and/or pressor therapy. Preferably the cleavage agent is a proteolytic enzyme, for example an endothelin converting enzyme.

Preferred enzymes are capable of cleaving Arg-Cys, Trp-Val, Trp-Ile, Ala-Ala bonds or cleaving a peptide immediately prior to an Arg-Arg dipeptide.

A sixth aspect of the present invention relates to a process for the preparation of a peptide of the first aspect, the process comprising coupling successive amino acid residues and/or coupling amino acid sequences together.

A seventh aspect of the present invention relates to a process for the preparation of a pharmaceutical composition of the third aspect, the process comprising admixing a peptide of the first aspect with a pharmaceutically acceptable carrier.

An eighth aspect of the present invention relates to a receptor molecule capable of binding an endothelin which is ET-1, ET-2 and/or ET-3 or a functional derivative thereof.

An ninth aspect of the present invention relates to a process for the preparation of a receptor molecule of the eighth aspect, the process comprising coupling successive amino acid residues and/or coupling amino acid sequences together.

Preferred features and characteristics of one aspect of the present invention are as for another *mutatis mutandis*.

The invention broadly relates to compositions and methods for regulating vascular activities. More specifically, the amino acid and nucleotide sequences of the peptides comprising the precursor forms of endothelin-2 (ET-2) and endothelin-3 (ET-3) are provided. Further, the invention concerns the purification and cloning of receptors for endothelin-1 (ET-1), ET-2, and ET-3 and the promotors ET-2 and ET-3. Methods for the uses of such compositions and therapy for human conditions are also disclosed.

The invention thus relates to compositions and methods for modulating vascular activities and for regulating growth and development of specific tissues, particularly tissues in which the compositions of the invention are expressed. Specifically, the preproendothelin forms of ET-2 and ET-3 are provided. ET-2 and ET-3 are vasoconstrictors in vitro and have been shown to increase blood pressure in vivo.

As indicated, ET-2 and ET-3 are members of a human endothelin family. So far, three structurally and pharmacologically distinct isopeptides have been isolated. In addition to the potent vasoconstrictor and pressor actions, endothelin has been reported to produce a wide spectrum of biological effects: regional vasodilatory effects in vivo; stimulation of proliferation of vascular smooth muscle cells and fibroblasts; contraction of airway and intestinal smooth muscle; positive inotropic and chronotropic effects on the myocardium; release of iconsanoids and/or endothelium-derived relaxing factor from vascular beds; stimulation of atrial natriuretic peptide secretion from atrial cardiocytes; inhibition of renin release from the glomerulus; and modulation of norepinephrine release from sympathetic terminals (Inoue et al., Proc. Natl. Acad. Sci. USA 86:2863-2867 (1989)). The family of endothelins appear to be similar to a group of peptide toxins from snake venom, the sarafotoxins.

Preproendothelin-2 comprises about 178 amino acids. The amino acid and nucleotide sequence is given in Figure 2. Preproendothelin-2 is capable of being cleaved to form ET-2. The gene encoding ET-2 has been localized to the short arm of chromosome 1. It is therefore not linked to ET-1 (chromosome 6) or the ET-3 gene.

The precursor form of ET-3 comprises approximately 238 amino acids which is capable of being cleaved to form ET-3. The amino acid and nucleotide sequence of prepro ET-3 is given in Figure 3. The gene encoding ET-3 has been localized to chromosome 20.

The tissue distribution of gene expression for ET-1, ET-2 and ET-3 is distinct. ET-2 mRNA is detected in human renal tissue, but not in human lung or umbilical vein endothelial cells (HUVEC). ET-3 is expressed in fetal lung, pancreas and spleen, and in the hypothalamus in adult and in human lung.

While the amino acid and nucleotide sequences of the prepro-forms are given, it is noted that variations of the sequences resulting in functional equivalents are encompassed by the invention.

The preproendothelin forms of ET-1, ET-2 and ET-3 share a homologous 15-amino acid sequence. This sequence contains eight amino acids also present in the first 15 residues of ET-2, including four cysteine residues in identical positions.

On the basis of the structures of the preproendothelin forms, the exons encoding ET-1, ET-2 and ET-3, as well as those encoding the ET-1-like and ET-3-like sequences, all evolved from a common ancestral exon. The ancestral exon probably duplicated to form exons encoding endothelin and endothelin-like sequences. Further, the ancestral gene containing these two exons replicated to form the three endothelin genes. Further support for the hypothesis that all six exons encoding endothelins and endothelin-like sequences arose from a common progenitor and that exon duplication preceded gene duplication is found in the observation that preproendothelin-2 contains an ET-2-like sequence and that this sequence lies in the carboxy terminal portion of the precursor.

The three endothelin-like sequences share six of 15 amino acids. Conservation of these sequences, as the genes diverged during evolution, suggests that they may have important yet unknown biological functions.

Because of the independent expression of the endothelin genes, the promoters regulating the expression of such genes are important for tissue-specific expression. Portions of the promoter regions for the preproendothelin-2 and preproendothelin-3 can be found in Figure 2 and Figure 3, respectively. Portions of these 5′ flanking regions can be utilized in hybridization experiments to isolate DNA fragments containing the promoter regions. See Maniatis et al., Molecular Cloning, A Laboratory Manual, Cold Spring Harbor Laboratories, Cold Spring Harbor, NY (1982) or Haymes et al., Nucleic Acid Hybridization, A Practical Approach, IRL Press, Washington, DC (1985). These promoter regions can be sequenced by conventional methods. See, for example, Sanger et al., Proc. Natl. Acad. Sci. USA 74:5463 (1977). Further, to determine the nucleotide sequences necessary for tissue-specific expression, deletion mutation experiments can be performed as described by Lee et al. J. Biol. Chem. 265:13432 (1990) and Thies et al., J. Biol. Chem. 265:10132.

Once promoter regions and necessary nucleotides have been identified, they can be used to regulate heterologous genes or heterologous promoters in a tissue-specific manner. In this manner, the promoters or nucleotide sequences thereof are operably linked to heterologous genes or heterologous promoters.

The pharmacologic activities of ET-1, ET-2 and ET-3 are quantitatively different, suggesting the existence of endothelin receptor subtypes. The present invention also concerns the purification, cloning and uses of the biological receptors for the endothelin genes.

The receptors of the present invention may be purified by routine adaptation of any of a variety of methods. In the method of Rosemblit et al., Endocrinol. 123:2284-2289 (1988), the receptors are isolated by a combination of affinity chromatography, lectin binding and SDS-polyacrylamide gel electrophoresis. See also, Nabel et al., Science 244:1342-1344 (1989); and Wilson et al., Science 244:1344-1346 (1989).

The identification of a preparation which contains a substantially purified receptor molecule permits the amino acid sequence of the receptor molecule to be determined, and further permits the molecule to be produced through the application of recombinant DNA technology. Thus, the amino acid sequences of the receptor molecules, the genetic sequences coding for the receptor molecules, the vehicles containing such genetic sequences, hosts transformed therewith, and receptor molecules produced through transformed host expression are encompassed by the present invention.

To obtain the amino acid sequence of the receptor molecules, the receptors in the substantially purified fractions are recovered by suitable methods known in the art. Affinity chromatography may be utilized as generally described by Rosemblit et al., supra, followed by concentration of sample using Centricon-30 (Amicon), and resolution by gel electrophoresis. The recovered molecules are then sequenced, preferably using an automated sequenator, and the amino acid sequence of the molecule thereby determined. Any suitable means can be used to determine the sequence of the receptor molecule. See, for example, Rodriguez, J. Chromatog. 350:217 (1985).

Once the amino acid sequence of the receptor peptide has been elucidated, one is able to isolate a gene sequence which is capable of encoding the entire receptor protein. In this manner, based upon the amino acid sequence, nucleotide probes are constructed which encode fragments of the receptor molecule. These probes can be utilized in hybridization experiments to isolate fragments containing genomic sequences encoding the receptor molecule.

The probability that a particular oligonucleotide will, in fact, constitute the actual receptor molecule fragment-encoding sequence can be estimated by considering abnormal base pairing relationships and the frequency with which a particular codon is actually used to encode a particular amino acid in eukaryotic cells. See Lathe et al., J. Molec. Biol. 183:1-12 (1985).

While the probe can be utilized to localize mRNA coding for the receptor molecules, to clone the gene sequence capable of encoding the receptor molecule, a DNA or, more preferably, a cDNA library is screened for its ability to hybridize with the oligonucleotide probes described above. The DNA probe is labeled with a detectable group, generally radiolabels such as $^3$H, $^{35}$S, $^{32}$P, $^{125}$I and $^{14}$C.

Alternatively, to clone a gene sequence which encodes the receptor molecules, a library of expression vectors is prepared by cloning DNA or, more preferably, cDNA (from a cell capable of expressing the receptor molecule) into an expression vector. The library is then screened for members capable of expressing a protein which binds to one of the endothelins. In this embodiment, DNA is extracted and purified from a cell which is capable of expressing the receptor molecule. The purified cDNA is fragmented to produce a pool of DNA fragments. The DNA fragments from this pool are then cloned into an expression vector in order to produce a genomic or cDNA library of expression vectors whose members each contain a unique cloned DNA or cDNA fragment.

Expression cloning utilizing a COS cell expression system may also be utilized. This system provides a powerful method to clone cDNAs coding for proteins with diverse functions. In particular, powerful methods have been derived for rapid cloning of cell surface markers and receptors. This approach involves the orchestration of several basic techniques in molecular biology. Included among these are construction of a cDNA expression library, transfection of plasmid DNA into eukaryotic cells, expression of transfected DNA, and selection and enrichment of a single cDNA clone. This technology provides a route by which one can study structure function relationships of receptor proteins without the laborious purification process often required for such proteins which are often present in very small numbers per cell. See, for example, Seed et al., Proc. Natl. Acad. Sci. 84:3365-3369 (1987); Wong et al., Science 228:810-815 (1985); Seed, B., Nature 329:840-842 (1987); Aruffo et al., EMBO J 6(11):3313-3316 (1987); Aruffo et al., Proc. Natl. Acad. Sci. USA 84:8573-8577 (1987); Simmons et al., Nature 331:624-627 (1988); Lee et al., Proc. Acad. Natl. Sci. USA 83:2061-2065 (1986); Yokota et al., Proc. Natl. Acad. Sci. USA 83:5894-5898 (1986); Yang et al., Cell 47:3-10 (1986).

For the most part, any vector for high level expression of proteins in eukaryotes may be utilized. Typical vectors include, but are not limited to, those described by Seed et al., Proc. Natl. Acad. Sci. 84:3365-3369 (1987); Wong et al., Science 228:810-815 (1985); Seed, B., Nature 329:840-842 (1987); and D'Andrea et al., Cell 57:277-285 (1989). Generally, any vector may be utilized which has the following features which make them suitable for COS cell expression cloning: 1) the eukaryotic transcription unit allows high-level expression in COS cells of a coding sequence; 2) the small size and SV40 elements allow high-level replication of the plasmid in COS cells; 3) the orientation of cloning sites provides for an efficient means of inserting cDNAs into the vector.

A high efficiency cDNA eukaryotic expression vector which directs high-level protein expression may be utilized to construct a cDNA library from a cell line known to express the endothelin receptors. In this manner, a cDNA library in the appropriate vector can be transfected into COS cells and individual cells selected which express high levels of single cDNAs.

The construction of a good expression library is the most important and probably the most difficult step in expression cloning. A number of general considerations for cDNA cloning are noted above, and are outlined in detail in a number of procedural manuals (Sambrook et al., Molecular cloning: A laboratory manual 2nd ed. (1989)). While the technology for construction of cDNA libraries is often difficult to master, the πHD and CDM8 vectors and kits for library construction are now commercially available. Several criteria need to be met by a library for expression cloning. The library should be developed from a cell line or tissue known to express the protein of interest, preferably at high levels. The library should have a large enough base so that there is good representation of all transcribed DNA. The cDNA inserts must be full or nearly full-length in order to express active protein.

The COS cell is the prototype cell for transient, high-level expression of transfected DNA (Gluzman, Y., Cell 23:175-182 (1981)). These cells were first developed by Gluzman from a simian kidney cell line which was stably transfected with replication origin defective SV40 virus. When these cells are transfected with a cloning vector containing the SV40 origin of replication, SV40 replicative machinery is utilized to increase the copy number of the construct inside the cell. This allows for a higher level of transient expression from the cDNA clone. COS cells appear capable of correct processing of various proteins, including proteins which are secreted, and proteins which insert into the plasma membrane and bind ligands. In general, COS cells are capable of transfection with more than one cDNA per cell and of producing $1 - 3 \times 10^4$ copies of each cDNA (Munro et al., Proc Natl Acad Sci USA 86:9248-9252 (1989)). They are thus capable of expressing on the order of $10^6$ copies of receptor protein per cell.

COS cells were derived from the CV-1 cell line which has a fibroblast morphology, but originated from cells isolated from the kidney. The cells clearly have a transformed phenotype. It is thus impossible to be certain whether COS cells will express a particular protein and thus be useless for an expression cloning strategy. It is necessary to screen the cells in any way possible to make certain that they do not express the gene to be cloned. In general, two COS cell lines have been commonly used (COS-1 and COS-7). Alternatively, a cell line derived from mouse fibroblasts which have been developed for expression cloning (WOP3027) may be utilized. (Seed, B., Nature 329:840-842 (1987)).

While most expression vectors are constructed in plasmids which contain the ampicillin or tetracycline resistance genes, those developed by Brian Seed for COS cell expression utilize a somewhat different strategy for selection in bacteria. The πH3 and CDM8 vectors must be grown in a strain of E. coli (designated MC1061/P3) for selection of antibiotic resistance. These bacteria carry a plasmid (p3) which encodes the ampicillin and tetracycline resistance genes. However, each has an amber mutation which prevents the expression of the resistance genes under normal conditions. The expression vectors contain a suppressor gene which allows both antibiotic resistance genes to be produced, and thus cells which also carry a πH3 or CDM8 expression plasmid will be ampicillin- and tetracycline-resistant.

The transfection of DNA into COS cells is a simple but important component of the cloning process. There are several techniques available for transfection. For example see Sambrook et al., Molecular cloning: A laboratory manual 2nd ed. (1989); Sompayrac et al., Proc. Natl. Acad. Sci. USA 12:7575-7578 (1981); Mulligan et al., Science 209:1422-1427 (1980). Two of the more commonly used techniques are DEAE dextran transfection and spheroplast fusion. Acceptable efficiency of transfection ranges from about 10% to about 30%. Once transfected, COS cells reach maximal protein accumulation in 60-70 hours. At this point a method of detection, selection, and enrichment for "positive" clones can be applied.

COS cells expressing a protein can be detected by assaying a measurable physiologic response (Wong et al., Science 228:810-815 (1985); Yang et al., Cell 47:3-10 (1986); Julius et al., Science 241:558-564 (1988)), or by virtue of ligand -receptor interaction. "Panning" on antibody-coated plates is an attractive means of screening for cell surface proteins since it allows for rapid enrichment of the clone of interest. This methodology requires that an antibody be available which is directed against the receptor or cell surface marker. However, an important extension of this technology has been the use of the ligand in the panning protocol.

It has been demonstrated by Staunton et al. that the panning methodology can be adapted for use with a ligand. For example, the use of LFA-1 to clone the receptor ICAM-2 (Staunton et al., Nature 339:61-64 (1989)). While these researchers adhered the ligand directly to the plastic dishes, alternatively, it may be advantageous to utilize biotin-labeled ligands and avidin-coated dishes in situations where there is no antibody available.

Another screening approach which allows rapid enrichment of positive clones is fluorescent sorting. On can bind fluorescent-labeled ligand to transfected COS cells and use cell sorting to isolate cells expressing the cell surface protein of interest (Yamasaki et al., Science 241:825-828 (1988)).

Another method of receptor cloning employs conventional binding assays to screen many small pools of cDNA clones (D'Andrea et al., Cell 57:277-285 (1989)); Sims et al., Science 241:585-589 (1988)). With this strategy, it is necessary to be able to detect binding to a small number of cells in the transfected pool.

Radio-labeled ligand binding to cells in culture, followed by autoradiography to detect and even isolate COS cells expressing the receptor may also be used (Munro et al., Proc. Natl. Acad. Sci. USA 86:9248-9252 (1989); Rattray et al., Mol. Brain Res. 7:249-250 (1990)). Receptors isolated by this technology have generally been detected with ligands of high affinity, $K_D$ less than 1 nanomolar, and radio-labeled to high-specific activity with $^{125}$I or [32]P. The utility of a detection system may be estimated by measuring detection of a positive control cell in a mixture of non-expressing cells (e.g., COS cells). A sensitive and specific means of detection allows one to proceed to selection and enrichment of COS cells expressing the cDNA encoding a receptor.

An expression screening strategy that is universally applicable is that of reduction cloning. In general, the expression library is divided into pools, and the pools screened to find evidence of a positive clone. The positive pool is then subdivided into smaller pools, and these are then evaluated with the same assay. Binding of radio-labeled ligand has been successfully used with this strategy to clone Gamma interferon, interleukin 1, and erythropoietin (Lee et al., Proc. Acad. Natl. Sci. USA 83:2061-2065 (1986); D'Andrea et al., Cell 57:277-285 (1989); Sims et al., Science 241:585-589 (1988)) receptors.

Ligand binding based methods have also been used to identify the biological activity of unknown receptor cDNAs (Bunzow et al., Nature 336:783-787 (1988); Dixon et al., Nature 321:75-79 (1986)). Ligands are generally labeled to high specific activity with $^{125}$I or[32]P. Low energy ligands labeled with $^{35}$S or $^3$H have not been useful due to their low specific activity and the practical need to use very high doses of these compounds in order to detect expressing COS cells. Due in part to the dynamic nature of ligand-receptor binding, very high affinity ligands are required, typically with dissociation constants of 100-1000 mol[-12]/l.

One unique aspect of reduction cloning is the need to divide a cDNA library into many small pools, grow the plasmids of each pool and prepare miniprep DNA (Sambrook et al., Molecular cloning: A laboratory manual 2nd ed. (1989)). The DNA of each miniprep is then transfected into COS cells, and the COS cells are screened for expression utilizing binding assays with radio-labeled ligand. Binding is measured by gamma counting or autoradiography. Pools which show binding are then divided into smaller pools and miniprep plasmid DNA is prepared. This second round of minpreps is then transfected into COS cells and binding is determined.

The receptor encoding cDNA is enriched with each cycle and the process of dividing-miniprepping-transfection-binding is repeated until a pure single cDNA carrying plasmid is isolated. Typically, 100-200 pools of 300-1,000 individual colonies are prepared so that 30,000-200,000 different cDNAs are screened. The size and number of pools may vary depending upon the rate of expression of a receptor in the cell or tissue from which a library was prepared as well as the affinity of ligand for receptor and the specific activity of radio-labeled ligand. Reduction cloning utilizing transfection of pooled clones and radiolabeled ligand binding is effective; however, it is very labor-intensive and requires the use of large quantities of radioligand.

Autoradiography has been applied recently to allow ligand-based detection while avoiding the need to divide a library into many pools. The murine interferon gamma receptor was isolated by this approach (Munro et al., Proc Natl Acad Sci USA 86:9248-9252 (1989)). Briefly, a cDNA library was constructed in πH3 and the complete library transfected into COS cells. After 2-3 days the cells were exposed to $^{32}$P-labeled murine-gamma-interferon, and then washed to remove unbound ligand. Autoradiography was used to detect receptor expressing cells and plasmid DNA was "rescued" by the Hirt supernatant procedure (Hirt B., J. Mol. Biol 26:365-369 (1967)). The rescued cDNA was amplified by transforming MC1061/P3 cells and plating on duplicate nitrocellulose filters. One copy of the filter was cut into pieces, the bacteria eluted, and plasmid prepped. Thus, small pools of clones were obtained.

Techniques such as, or similar to, those described above have successfully enabled the cloning of genes for human aldehyde dehydroxygenases (Hsu et al., Proc. Natl. Acad. Sci. USA 82:3771-3775 (1985)), fibronectin (Suzuki et al., Eur. Mol. Biol. Organ. J. 4:2519-2524 (1985)), the human estrogen receptor gene (Walter et al., Proc. Natl. Acad. Sci. USA 82:2889-2893 (1985)), tissue type plasminogen activator (Pennica et al., Nature 301:214-221 (1983)), and human term placental alkaline phosphatase complementary DNA (Kam et al., Proc. Natl. Acad. Sci. USA 82:8715-8719 (1985)).

The invention also encompasses functional derivatives of the receptor molecules. The term "functional derivative" is intended to include fragments, variants, analogs, or chemical derivatives of the receptor molecules. The term "fragment" is meant to refer to any polypeptide subset of the molecule. The term "variant" is meant to refer to a molecule substantially similar in structure and function to either the entire molecule or a fragment thereof, and may differ from the latter by deletion, addition or substitution of one or more amino acids. A molecule is said to be substantially similar if both molecules have substantially similar structures or if both molecules possess a similar biological activity. The term "analog" is meant to refer to a molecule substantially similar in function to either the entire molecule or a fragment thereof. As used herein, a molecule is said to be a chemical derivative of another molecule when it contains additional chemical moieties not normally a part of the molecule. Such moieties may improve the molecule's solubility, absorption, half-life, etc. The moieties may alternatively decrease the toxicity of the molecule, eliminate or attenuate any undesirable side-effect of the molecule, and the like. Moieties capable of mediating such effects are disclosed in Remington's Pharmaceutical Sciences (1980), the disclosure of which is herein incorporated by reference.

Once the receptor molecules have been elucidated, agonists or antagonists which bind to the receptor molecules can be identified. These agonists and antagonists can be utilized to mediate a physiologically significant effect; for example, competing with the endothelin molecules for the receptor to lower blood pressure or to alter other vascular functions. Antibodies which bind the receptor molecule can also be utilized for the same effect. Methods are known in the art for the production of antibodies and monoclonal antibodies. These include Kohler et al., Nature 265:495 (1975); Kohler et al., Eur. J. Immunol. 6:511 (1976); Kohler et al., Eur. J. Immunol. 6:292 (1976); Klein, J., Immunology: The Science of Cell-Noncell Discrimination, John Wiley & Sons, New York (1982); Campbell, A., "Monoclonal Antibody Technology, in Laboratory Techniques in Biochemistry and Molecular Biology, Vol. 13, Burdon et al. (eds.), Elsevier, Amsterdam (1984); as well as U.S. Patent Nos. 4,609,893; 4,713,325; 4,716,111; 4,716,117; and 4,720,459.

The invention will now be described by way of example, and not limitation, with reference to the accompanying drawings in which:

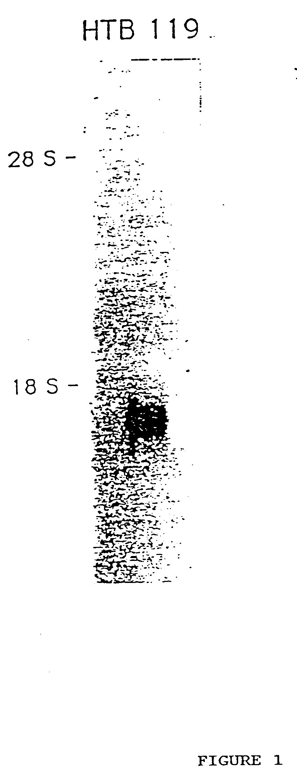

Figure 1. Identification of ET2 mRNA in HTB119 cells by RNA blot hybridization. Expression of the ET2 gene was identified in the HTB119 cell line derived from a human small cell carcinoma. Total cellular RNA, 15 μg, was fractionated on a formaldehyde agarose gel and transferred to a nitrocellulose filter. After hybridization to a radiolabeled 192-bp fragment of the ET2 gene, the RNA blot was washed under stringent conditions and exposed to x-ray film for 24 hours. The migration of ribosomal RNA is shown (28 S and 18 S).

Figure 2. Nucleotide and translated amino acid sequence of the ET2 cDNA. The ET2 cDNA isolated from the HTB119 cDNA library is 1261 nucleotides long. A 534-nucleotide open reading frame encoding the 178-amino acid precursor, preproendothelin 2, follows the ATG codon closest to the 5′ end. The amino acid sequence of ET2 (residues 49 to 69) is underlined. A 15-amino acid sequence sharing homology with ET2, the ET2-like sequence (residues 96 to 110), is indicated by a dashed underline. Amino acids of the probable leader segment are shown in capital letters, and the predicted site of leader segment cleavage (between residues 26 and 27) is indicated by an arrow. Amino acid sequences are numbered to the left, and nucleotide sequences are numbered in parentheses.

Figure 3. Nucleotide and translated amino acid sequences of the composite endothelin 3 cDNA. A 714-nucleotide open reading frame encodes a 238-amino acid precursor, preproendothelin 3. The amino acid sequence of ET3 (preproendothelin 3 residues 97-117) is indicated by a continuous line. The ET3-like sequence, a 15-amino acid sequence (preproendothelin 3 residues 159-173) homologous to ET3, is indicated by a dashed line. Amino acids of the probable leader segment are in capital letters. The arrow indicates the probable site of leader segment cleavage (between Ser25 and Gly26). Amino acid sequences are numbered at the left, nucleotide sequences are numbered in parentheses. (The cDNA inserts were incomplete but overlapping. One cDNA insert encoded nucleotides 1-1287, the other encoded nucleotides 318-2233.)

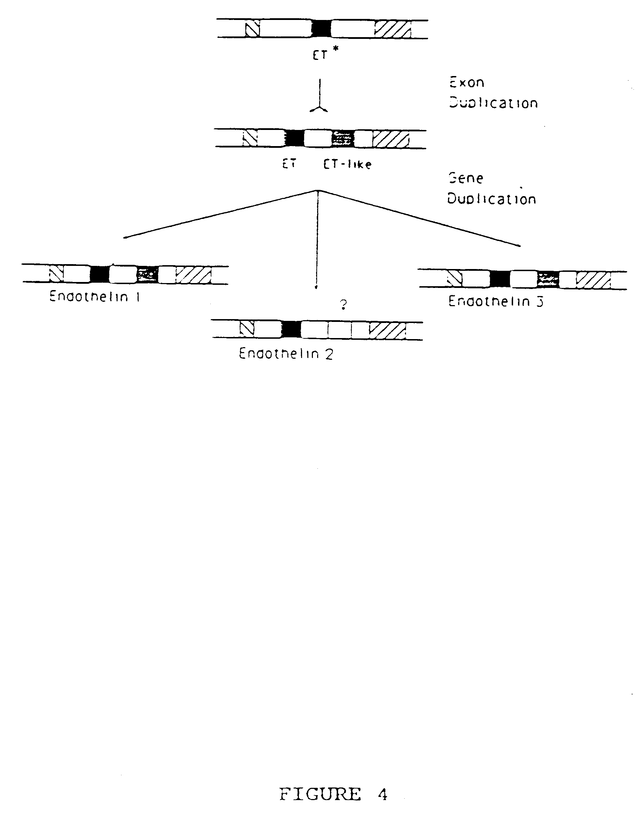

Figure 4. Evolution of the endothelin gene family. An ancestral exon encoding an endothelin-related peptide (ET*) probably duplicated to form exons specifying endothelin (ET) and endothelin-like (ET-like) sequences.

Subsequent gene duplication events most likely led to formation of the three endothelin genes. The exons encoding ET1, ET2, and ET3, as well as those specifying the ET1- and ET3-like sequences, probably descended from a single ancestral exon. The question mark indicates a hypothetical exon encoding the ET2-like sequence.

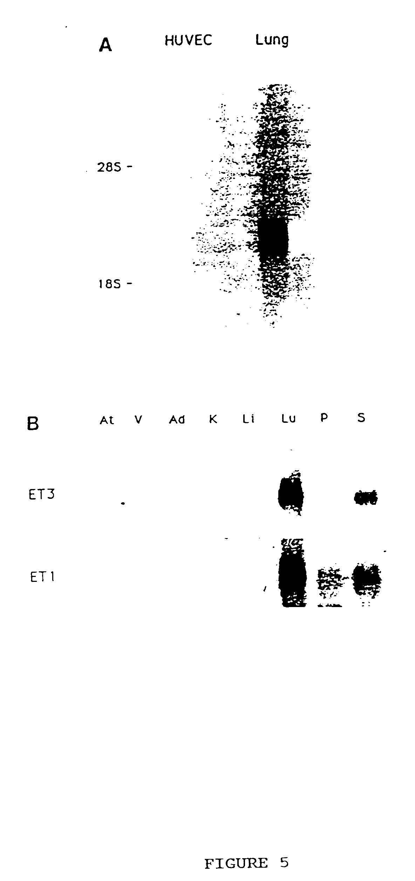

Figure 5. Identification of ET3 and ET1 mRNAs by RNA blot hybridization. In A, expression of the ET3 gene was investigated in cultured human umbilical vein endothelial cells (HUVEC) and fetal human lung tissue. A 1.3-kb Pst1-EcoRI restriction fragment containing the 3'-untranslated portion of the ET3 cDNA was used to specifically detect ET3 mRNA. After hybridization, the RNA blot was exposed to x-ray film for 24 hours. The migration of ribosomal RNA is indicated (28S and 18S). The ET3 gene transcript was approximately 2500 nucleotides long. In B, levels of ET3 and ET1 mRNA are compared in human fetal tissues (At, atria; V, ventricles; Ad, adrenals; K, kidney; Li, liver; Lu, lung; P, pancreas; S, spleen). An RNA blot was prepared from human fetal tissues and hybridized with the ET3 cDNA restriction fragment. The filter was exposed to x-ray film for 48 hours. Prolonged exposures to x-ray film permitted clear visualization of low levels of ET3 mRNA in kidney tissue (data not shown). Radiolabeled DNA was then removed from the filter as described under "Experimental Procedures." The filter was hybridized with a 0.9-kb EcoRI restriction fragment derived from the ET1 cDNA which specifically detected ET1 mRNA. The filter was subjected to autoradiography for 48 hours.

The following Examples are presented by way of example and illustration and are not to be construed as limiting on the present invention.

## Example 1

Cell line. The HTB119 cell line was obtained from the American Type Culture Collection (Rockville, MD). Cells were maintained in RPMI 1640 with 10% fetal bovine serum.

Isolation and radiolabeling of endothelin 2 gene probe. A 192-bp fragment of the ET2 gene was amplified the polymerase chain reaction (Sambrook et al., Molecular Cloning, A Laboratory Manual, Cold Spring Harbor Laboratory Press, Cold Spring Harbor, New York (1989)). Human genomic DHA was incubated with Taq polymerase (united States Biochemical, Cleveland, OH) and two oligonucleotides, 5,-GGGAATTCAAGGGCCAGGCTGCTGCCAC-3' [Seq. ID No: 1] and 5'-GGGAATTCAGCTCCCCCTGCCCCCAATC-3' [Seq. ID No: 2]. The first oligonucleotide contains nucleotides 1 to 20 of the endothelin 2 genomic fragment described by Inoue et al. (Inoue et al., Proc. Natl. Acad. Sci. USA 86:2863-2867 (1989)), and the second oligonucleotide is complementary to nucleotides 172 to 192. The reaction mixture was subjected to 40 cycles of heat denaturation (94°C, 2 min), annealing (37°C, 2 min), and extension (72°C, 2 min). The amplified ET2 gene fragment was subcloned into the EcoRI site of pUC13.

The ET2 gene probe was radiolabeled by including $\alpha$-[$^{32}$P]dCTP in the polymerase chain reaction (Schowalter et al., Anal. Biochem. 177:90-94 (1989)). Briefly, the EcoRI restriction fragment containing the ET2 gene probe was mixed with the oligonucleotides described above, Taq DHA polymerase, $\alpha$-[$^{32}$P]dCTP, and non-radioactive dGTP, dATP, and dTTP. The reaction mixture was subjected to 20 cycles of heat denaturation (94°C, 1 min), annealing (50°C, 2 min), and extension (72°C, 3 min). Radiolabeled fragment was separated from unincorporated nucleotides by centrifugation through a G-50 Sephadex spin column.

RNA blot hybridization. Cellular RNA was prepared by quanidinium isothiocyanate extraction and centrifugation through cesium chloride (Sambrook et al., Molecular Cloning, A Laboratory Manual, Cold Spring Harbor Laboratory Press, Cold Spring Harbor, New York (1989)). RNA was fractionated on a 1.3% formaldehyde-agarose gel and transferred to nitrocellulose filters. Filters were hybridized in the presence of $^{32}$P-labeled ET2 gene probe at 42°C, washed in 3 mM sodium citrate, 30 mM sodium chloride, and 0.1% sodium dodecyl sulfate at 65°C, and exposed to KODAK XAR-5 film.

Cloning endothelin 2 cDNA. Cellular was extracted from HTB119 cells, as described above, and Poly-A$^+$ was prepared (Sambrook et al., Molecular Cloning, A Laboratory Manual, Cold Spring Harbor Laboratory Press, Cold Spring Harbor, New York (1989)). The first strand of cDNA was synthesized by using oligo(dT) in the presence of reverse transcriptase (Sambrook et al., Molecular Cloning, A Laboratory Manual, Cold Spring Harbor Laboratory Press, Cold Spring Harbor, New York (1989)). Second strand cDNA synthesis was performed with E. coli DNA polymerase 1 and E. coli RNase H. Double-stranded cDNA was incubated with T4 DNA polymerase, methylated with EcoRI methylase, and ligated with EcoRI linkers. After digestion with EcoRI restriction endonuclease, cDNA was fractionated on a 1% SeaPlaque agarose gel (FMC Bioproducts, Rockland, MD). cDNA, approximately 0.7 to 5 kb in size, was extracted from the gel, ligated into λGT11, and incubated in the presence of phage packaging extract (Promega). Bacteriophages were plated and transferred to Biotrace nylon membranes (Gelman). Membranes were hybridized with the gene probe as described for blot hybridization. Bacteriophage that hybridized with the radiolabeled probe were isolated by plaque purification, and DNA was prepared from phage stocks.

Chromosomal mapping. Southern blots were prepared (Naylor et al., J. Exp. Med. 57:1020-1027 (1983)) from Hind3-digested DNA from 45 human-mouse somatic hybrid cell lines. Blots were hybridized with the $^{32}$P-labeled ET2 gene probe, as described for RNA blot hybridization. The hybrids were derived from 17 unrelated human cell lines and 4 mouse cell lines (Shows et al., In Advances in Human Genetics, H. Harris and K. Hirschnorn, eds., Plenum, New York and London. Vol. 12, pp. 341-452 (1982); Shows et al., Somat. Cell Mol. Gen. 10:315-318 (1984); Shows et al., Cytogenet. Cell Genet. 21:99-104 (1978)) and were characterized by karyotypic analysis, by mapped enzymye markers, and by mapped DNA probes (Shows et al., In Advances in Human Genetics, H. Harris and K. Hirschnorn, eds., Plenum, New York and London. Vol. 12, pp. 341-452 (1982); Shows et al., Cytogenet. Cell Genet. 21:99-104 (1978); Shows et al.. In Isozymes: Current Topics in Biological and Medical Research, M. C. Rattazzi et al., eds., Alan R. Liss, New York. Vol. 10, pp. 323-339 (1983)).

## Results and Discussion

Identification of a cell line expressing the endothelin 2 gene. To isolate and characterize a cDNA clone complementary to ET2 mRNA, we required a source of ET2 mRNA. RNA blot hybridization with a 192-bp fragment of the human ET2 gene was used to detect ET2 in human cell lines and tissues. The 192-bp ET2 gene probe was amplified from human genomic DNA using the Taq DNA polymerase and two flanking oligonucleotides complementary to the sequence of the ET2 genomic fragment isolated by Inoue et al. (Inoue et al., Proc. Natl. Acad. Sci. U.S.A. 86:2863-2867 (1989)). The ET2 gene fragment was radiolabeled by the polymerase chain reaction with $\alpha$-[32P]dCTP included in the reaction mixture. The HTB119 cell line, derived from a human small cell lung carcinoma, was observed to contain mRNA hybridizing to the ET2 gene probe under stringent washing conditions (Fig. 1). By comparison with 28 S and 18 S ribosomal RNA, the length of the hybridizing mRNA was approximately 1400 nucleotides, distinguishing it from the ET1 and ET3 mRNAs, which are both greater than 2000 nucleotides in length (Itoh et al., FEBS Lett. 231:440-444 (1988); Bloch et al., J. Biol. Chem. 264:18156-18161 (1989)). ET2 was also detected in the COS-7 cell line derived from monkey kidney. Low levels of ET2 were detected in fetal human kidney, but not in human umbilical vein endothelial cells (passage number 4), or in fetal lung, spleen, liver, or adrenal gland (data not shown).

Cloning the endothelin 2 cDNA. To characterize the ET2 precursor and to confirm that the 1.4-kb mRNA hybridizing to the ET2 gene probe was the ET2 gene transcript, we cloned the ET2 cDNA. Poly-A+ RNA was extracted from HTB119 cells and cDNA was prepared and cloned into $\lambda$GT1I. The cDNA library was screened with the radiolabeled ET2 gene probe: one of approximately $10^6$ bacteriophages hybridized to the probe. DNA was prepared from this bacteriophage, and the nucleotide sequence of the cDNA insert was determined (Fig. 2).

The translated cDNA sequence predicts that the encoded precursor is 178 amino acids in length and includes the 21-amino acid sequence of ET2. It is likely that the deduced amino acid sequence derived from this clone represents the entire coding region, with the initial methionine representing the start of translation. The nucleotide sequence surrounding the first ATG codon (nucleotides 71-73) corresponds to sequences that are important in determining the translation start site of other eukaryotic genes (Kozak, Cell 44:283-292 (1986)). The amino terminus of the precursor contains hydrophobic amino acids characteristic of a signal sequence, and the probable signal sequence cleavage site is between residues Ala[26] and Ala[27] (von Heijne, Eur. J. Biochem. 133:17-21 (1983)). ET2 is flanked at its amino terminus by an arginine dipeptide, a potential cleavage site for processing enzymes. The next pair of arginine residues is at positions 86 and 87; cleavage prior to these arginines may generate a 36-amino acid "big ET2". Subsequent proteolysis of big ET2 between residues Trp[69] and Val[70], by an "endothelin converting enzyme," would yield mature ET2. Similar post-translational processing has been proposed for the maturation of ET1 and ET3 precursors (Inoue et al., Proc. Natl. Acad. Sci. U.S.A. 86:2863-2867 (1989); Yanagisawa et al., Nature 332:411-415 (1988); Bloch et al., J. Biol. Chem. 264:18156-18161 (1989)).

Near its carboxyl terminus, preproendothelin 2 shares with the ET1 and ET3 precursors a 15-amino acid sequence homologous with ET2. This ET2-like sequence contains eight amino acids also present in the first fifteen residues of ET2, including four cysteine residues in identical positions. Previous studies of the chromosomal organization of the ET1 gene revealed that ET1 and the ET1-like sequence are encoded on separate exons, which probably arose as a result of exon duplication (Bloch et al., J. Biol. Chem. 264:10851-10857 (1989)). On the basis of the structures of preproendothelin 1 and 3, the organization of the ET1 gene, and the partial characterization of the ET2 and ET3 genes (Inoue et al., Proc. Natl. Acad. Sci. U.S.A. 86:2863-2867 (1989)), we previously suggested that exons encoding ET1, ET2, and ET3, as well as those encoding the ET1-like and ET3-like sequences, all evolved from a common ancestral exon (Bloch et al., J. Biol. Chem. 264:18156-18161 (1989)). We proposed that the ancestral exon duplicated to form exons encoding endothelin and endothelin-like sequences and that the ancestral gene containing these two exons replicated to form the three endothelin genes. However, it remained plausible that gene duplication preceded exon duplication and that exons specifying ET1-like and ET3-like sequences arose as a result of two independent exon duplication events. The observations that preproendothelin 2 contains an ET2-like sequence and that this sequence lies in the carboxyl terminal portion of the precursor support our hypothesis that all six exons encoding endothelins and endothelin-like sequences arose from a common progenitor and that exon duplication preceded gene duplication.

The three endothelin-like sequences share six of fifteen amino acids. Conservation of these sequences, as the genes diverged during evolution, suggests that they may have important, as yet unknown, biological functions.

Chromosomal assignment. Members of a gene family arising as a result of gene duplication are frequently clustered on the same chromosome (Lewin, Genes, John Wiley and Sons, New York, Chapter 21, pp. 341-356 (1985)). We previously assigned the loci containing the ET1 gene, designated EDN1 (McAlpine et al., Cytogenet. Cell. Genet. 51:13-66 (1989)), and that containing the ET3 gene, designated EDN3, to chromosomes 6 and 20, respectively (Bloch et al., J. Biol. Chem. 264:18156-18161 (1989); Bloch et al., J. Biol. Chem. 264:10851-10857 (1989)). To determine whether the locus containing the ET2 gene, designated EDN2, is closely linked to either EDN1 or EDN3, DNA isolated from human-mouse somatic hybrid cell lines was used to assign the ET2 gene to a human chromosome. Southern blots prepared with Hind3-digested DNA from hybrid cell lines were hybridized with the 192-bp ET2 gene probe. The ET2 gene probe hybridized with an approximately 6.3-kb human Hind3 restriction fragment and with murine fragments of approximately 8.5 and 15 kb (data not shown). Detection of human sequences hybridizing with the ET2 gene probe correlated with the presence of human chromosome 1 (Table I). Four hybrid cell lines contained translocations of chromosome 1, but not intact chromosome 1. The ET2 gene probe hybridized with DNA from hybrid cell lines JWR-22H (2pter->2q37::1p21->1pter) and XOL-6 (1pter->1q12::Xq26->Xqter) but not with DNA from JWR-26C (1p21->1qter::2q37->2qter) and XOL-9 (Xpter->Xq26::1q12->1qter). EDN2 is therefore further localized to the 1p21->1pter region of human chromosome 1. Assignment of EDN2 to chromosome 1 demonstrates that the closely-related endothelin genes are not linked. The genes apparently dispersed after gene duplication. Localization of endothelin genes on human chromosomes may in the future permit linkage of these genes with human genetic disorders.

Detection and characterization of transcripts of the ET2 gene in a human cell line and, at low levels, in fetal human kidney demonstrates that the ET2 genomic fragment identified by Inoue et al. (Inoue et al., Proc. Natl. Acad. Sci. U.S.A. 86:2863-2867 (1989)) is a portion of a transcriptionally active gene and not a pseudogene. The tissue distribution of gene expression is distinct for each of the endothelin genes. In particular, neither the ET2 nor ET3 gene is expressed in human umbilical vein endothelial cells, whereas the ET1 gene is expressed at high levels. These observations suggest that the three genes have different physiological functions. Although it is possible that ET2 and BT3 genes are expressed in endothelial cells at other sites, it seems likely that these genes have biological roles outside of the vasculature.

Identification of cell lines transcribing the ET2 gene should permit investigation of the regulation of ET2 gene transcription, as well as ET2 synthesis and posttranslational processing. Comparison of ET1 biosynthesis in endothelial cells and ET2 biosynthesis in HTBl19 cells may offer insights into the cellular mechanisms regulating their production.

Table I.  Chromosomal assignment of the locus for the ET2 gene, EDN2:  Segregation of human chromosomes with sequences hybridizing to the ET2 gene probe in human-mouse cell hybrid cell lines

| Chromosome | Concordant # of Hybrids | | Discordant # of Hybrids | | % Discordancy |
|---|---|---|---|---|---|
| | (+/+) | (-/-) | (+/-) | (-/+) | |
| 1 | 13 | 28 | 0 | 0 | 0 |
| 2 | 10 | 19 | 4 | 11 | 34 |
| 3 | 13 | 14 | 2 | 14 | 37 |
| 4 | 10 | 18 | 5 | 12 | 38 |
| 5 | 12 | 19 | 3 | 11 | 31 |
| 6 | 10 | 21 | 5 | 9 | 31 |
| 7 | 12 | 19 | 2 | 10 | 28 |
| 8 | 13 | 17 | 2 | 13 | 33 |
| 9 | 6 | 26 | 8 | 3 | 26 |
| 10 | 15 | 13 | 0 | 17 | 38 |
| 11 | 9 | 19 | 4 | 11 | 35 |
| 12 | 15 | 18 | 0 | 12 | 27 |
| 13 | 9 | 19 | 6 | 11 | 38 |
| 14 | 14 | 10 | 1 | 20 | 47 |
| 15 | 12 | 19 | 2 | 11 | 30 |
| 16 | 8 | 24 | 7 | 6 | 29 |
| 17 | 13 | 7 | 1 | 22 | 53 |
| 18 | 13 | 12 | 2 | 18 | 44 |
| 19 | 11 | 23 | 4 | 7 | 24 |
| 20 | 10 | 13 | 5 | 17 | 49 |
| 21 | 11 | 8 | 4 | 22 | 58 |
| 22 | 10 | 20 | 4 | 8 | 29 |
| X | 7 | 8 | 3 | 19 | 59 |

Southern blots containing Hind3-digested DNA from 45 human-mouse cell hybrid cell lines were hybridized with the ET2 gene probe. Scoring for the human ET2 gene was determined by the presence (+) or absence (-) of a human 6.5-kb restriction fragment that hybridized with the probe. The scoring was compared with the presence or absence of human chromosomes in each hybrid. A 0% discordancy indicates a matched segregation of EDN2 with a chromosome and is the basis for chromosome assignment. EDN2 mapped to human chromosome 1.

**Example 2**

Experimental Procedures

Cloning the Endothelin 3 Gene. A 1.2-kb EcoRI restriction fragment prepared from a human ET1 cDNA was used to screen a library derived from human leyukocyte DNA that had been incompletely digested with the restriction endonuelcase MboI and ligated into the bacteriophage EMBL-3 (Clontech Laboratory, Palo Alto, CA). The restriction fragment was labeled with DNA polymerase I in the presence of random sequence hexanucleotides and [$\alpha$-$^{32}$P]dCTP. The bacteriophage library was plated, and nitrocellulo;se filters were prepared. Filters were incubated with the radiolabeled restriction fragment at 37°C as described (Strauss, W.M., in Current Protocols in Molecular Biology, Ausubel, F.M., et al., (eds.), Unit 6.3, Greene Publishing Associates and Wiley-Interscience, New York, pp. 1-6 (1987)). Filters were washed in 3 mM sodium citrate, 30 mM sodium chloride, and 0.1% sodium dodecyl sulfate at room temperature (nonstringent washing conditions) and were exposed to Kodak XAR-5 film overnight at -70°C. Subsequently, filters were washed in the same solution at 65°C (stringent washing conditions), and autoradiography was repeated. Bacteriophage hybridizing with the ET1 cDNA probe after nonstringent washing, but not after stringent washing, were isolated by plaque purification. DNA was prepared from phage stocks according to standard techniques. Nucleotide sequence determination (see below) demonstrated that these genomic clones contained portions of the ET3 gene.

Cloning Endothelin 3 cDNAs. A 0.5 kb EcoRI-BglII restriction fragment derived from the ET3 gene was used to screen a cDNA library that had been prepared from adult human hypothalamic mRNA and cloned into the bacteriophage λGT11 (kindly provided by Dr. Richard H. Goodman, New England Medical Center, Boston). The restriction fragment was labeled with DNA polymerase I in the presence of random hexanucleotides and [α-$^{32}$P]dCTP. Bacteriophages were plated and transferred to nitrocellulose filters. After hybridization to the $^{32}$P-labeled EcoRI-BglII restriction fragment at 42°C, the filters were washed under stringent conditions and subjected to autoradiography. Bacteriophages that hybridized with the EcoRI-BglII restriction fragment were isolated by plaque purification, and DNA was prepared from phage stocks.

Sequencing the ET3 cDNA and Gene. Restriction fragments derived from bacteriophages containing the ET3 cDNA and gene were sequenced by the dideoxynucleotide chain termination procedure, with modified T7 DNA polymerase (United States Biochemical Corp., Cleveland, OH). DNA fragments were ligated into the plasmid vector pUC13 and into M13 phage vectors mp18 and mp19. Sequencing primers were M13 "universal" primer (United States Biochemical), an oligonucleotide derived from nucleotides 1649-1666 of the ET3 cDNA, an oligoncueltodie complementary to nucleotides 1802-1819 of the ET3 cDNA, and an oligonucleotide complementary to nucleotides 175-219 of the porcine endothelin cDNA (Yanagisawa, M., et al., Nature 332:411-415 (1988)).

Chromosomal Mapping. Southern blots were prepared (Naylor, S.L., et al., J. Exp. Med. 57:1020-1027 (1983)) from HindIII-digested DNA from 36 human-mouse somatic hybrid cell lines. Blots were hybridized with the $^{32}$P-labeled EcoRI-BglII restriction fragment of the ET3 gene. The hybrids were derived from 13 unrelated human cell lines and 4 mouse cell lines (Shows, T.B., et al., in Advances in Human Genetics, Harris, H., et al., (eds.), Plenum Publishing Corp., New York, Vol. 12, pp. 341-452 (1982); Shows, T., et al., Somatic Cell Mol. Genet. 10:315-318 (1984); Shows, T.B., et al., Cutogenet. Cell Genet. 21:99-104 (1978)) and were characterized by karyotypic analysis, by mapped enzyme markers, and by mapped DNA probes (Shows, T.B., et al., in Advances in Human Genetics, Harris, H., et al., (eds.), Plenum Publishing Corp., New York, Vol. 12, pp. 341-452 (1982); Shows, T.B., et al., Cutogenet. Cell Genet. 21:99-104 (1978); Shows, T.B., in Isozymes: Current Topics in Biological and Medical Research, Rattazzi, M.C., et al. (eds.), Vol. 10, pp. 323-339, Alan R. Liss, New York (1983)). Blots were incubated in the presence of the radiolabeled restriction fragments at 42°C, washed under stringent conditions, and autoradiographed as described (Strauss, W.M., in Current Protocols in Molecular Biology, Ausubel, F.M., et al., (eds.), Unit 6.3, Greene Publishing Associates and Wiley-Interscience, New York, pp. 1-6 (1987)).

RNA Blot Hybridization. Cellular RNA was prepared by guanidinium isothiocyanate extraction and centrifugation through cesium chloride (Kingston, R.E., in Current Protocols in Molecular Biology, Ausubel, F.M., et al. (eds.), Unit 4.2, pp. 1-5, Greene Publishing Associates and Wiley-Interscience, New York (1987)). RNA was fractionated on a 1.3% formaldehyde-agarose gel and transferred to Nitroplus 2000 nylon membranes (Micron Separations, Inc., Westboro, MA). Hybridization probes were a 1.3-kb PstI-EcoRI restriction fragment containing the 3'-untranslated portion of the ET3 cDNA (Fig. 3; nucleotides 921-2233) and a 0.9-kb EcoRI restriction fragment containing the 3'-untranslated portion of the ET1 cDNA (Bloch, K.D., et al., J. Biol. Chem. 264:10851-10857 (1989)). Filters were hybridized with $^{32}$P-labeled probes at 42°C, washed in 3 mM sodium citrate, 30 mM sodium chloride, and 0.1% sodium dodecyl sulfate at 60°C, and exposed to Kodak XAR-5 film. When required, radiolabeled DNA was removed from filters by incubation in 50% formamide, 10 mM Tris-HCl, and 1 mM EDTA at 80°C for 15 min, and removal of the probe was documented by autoradiography. There was no nucleotide sequence homology between restriction fragments derived from the 3'-untranslated regions of the two cDNAs, nor did they cross-hybridize under the test conditions.

Results and Discussion

Isolation of the Endothelin-3 Gene. To identify genes related to the ET1 gene, a 1.2 kb EcoRI restriction fragment prepared from a human ET1 cDNA (Bloch, K.D., et al., J. Biol. Chem. 264:10851-10857 (1989)) was used to screen an EMBL-3 bacteriophage library prepared from human leukocyte DNA (Clontech Laboratory). Approximately 1 x 10$^6$ bacteriophages were plated and transferred to nitrocellulose filters. After hybridization with the $^{32}$P-labeled cDNA restriction fragment, the filters were washed under nonstringent conditions and subjected to autoradiography. The filters were then washed under stringent conditions and autoradiography was repeated. Four bacteriophages were isolated which hybridized with the ET1 cDNA after nonstringent washing but not after stringent washing. DNA from the four bacteriophages was examined by restriction enzyme DNA mapping, and the four bacteriophages were found to have similar genomic inserts. Southern blot analysis of DNA from one of the bacteriophages revealed that sequences hybridizing to the ET1 cDNA could be localized to a 0.5-kb EcoRI-BglII restriction fragment. Nucleotide sequence determination of this restriction fragment (data not shown) revealed sequences identical to those of the ET3 genomic clone isolated by Inoue et al. (Inoue, A., et al., Proc. Natl. Acad. Sci. USA 86:2863-2867 (1989)).

Isolation of Endothelin 3 cDNAs. The 0.5-kb EcoRI-BglII restriction fragment of the ET3 gene was used to screen a cDNA library that had been prepared from adult human hypothalamic mRNA and cloned into the bacteriophage λGT11 (kindly provided by Dr. Richard H. Goodman, New England Medical Center, Boston). Approximately $1.5 \times 10^6$ bacteriophages were plated and transferred to nitrocellulose filters. After hybridization to the $^{32}$P-labeled EcoRI-BglII restriction fragment, the filters were washed under stringent conditions and subjected to autoradiography. Two bacteriophages hybridized with the genomic fragment. DNA was prepared from the bacteriophages, and the nucleotide sequences of the two cDNA inserts were determined.

Although neither cDNA insert is full-length, they are overlapping. The composite cDNA sequence (Figure 3) includes an open reading frame of 714 base pairs. An in-frame, TGA termination codon is located 12 base pairs upstream of the open reading frame. The encoded peptide includes a 21-amino acid sequence that shares 15 of 21 amino acids with ET1, including four cysteines occupying corresponding positions. This 21-amino acid sequence is identical to that of a peptide previously identified as rat endothelin (Yanagisawa, M., et al., Proc. Natl. Acad. Sci. USA 85:6964-6967 (1988)) and as human ET3 (Inoue, A., et al., Proc. Natl. Acad. Sci. USA 86:2863-2867 (1989)). Nucleotides 344-558 of the composite cDNA are identical to the sequence of the ET3 gene exon reported by Inoue et al. (Inoue, A., et al., Proc. Natl. Acad. Sci. USA 86:2863-2867 (1989)).

The $NH_2$ terminus of the 238-amino acid precursor of ET3, preproendothelin 3, contains hydrophobic amino acids characteristic of a signal sequence (Fig. 3). Based on the method of prediction proposed by von Heijne (von Heijne, G., Eur. J. Biochem. 133:17-21 (1983)), the signal sequence cleavage site is probably between residues $Ser^{25}$ and $Gly^{26}$. ET3 is flanked at its $NH_2$ terminus by an arginine dipeptide, a potential cleavage site for processing enzymes. At its COOH end, ET3 shares with ET1 a tryptophan residue followed by a hydrophobic residue. Yanaagisawa et al. (Yanagisawa, M., et al., Nature 332:411-415 (1988)) proposed that the unusual proteolysis between tryptophan and valine that generates mature ET1 is accomplished by an endothelin-converting enzyme. The same or a similar enzyme may participate in the processing of ET3. Analogous to the hypothetical intermediate big ET1, big ET3, formed by cleavage at pairs of basic amino acids (preproendothelin 3 residues 95 and 96 and 139 and 140), may represent an intermediate in ET3 biosynthesis.

Preproendothelin 3 is similar to its ET1 counterpart in that it contains a 15-amino acid sequence that has homology with ET3. This ET3-like sequence shares six amino acids with ET3, and the cysteine residues are in identical positions. ET3 and ET3-like sequences are encoded on separate exons (Inoue, A., et al., Proc. Natl. Acad. Sci. USA 86:2863-2867 (1989); and data not shown). Previously, we suggested that exons encoding ET1 and the ET1-like sequence arose by means of exon duplication (Bloch, K.D., et al., J. Biol. Chem. 264:10851-10857 (1989)). By a similar mechanism, exons specifying ET3 and ET3-like sequences are likely to represent descendants of a common progenitor exon. Furthermore, since it is probable that ET1, ET2 and ET3 genes are the product of gene duplication events, exons encoding the three endothelins and the two known endothelin-like sequences may all derive from a single ancestral exon (Fig. 4). Because of the similarity of the 3′ ends of the exons encoding the three endothelins (Inoue, A., et al., Proc. Natl. Acad. Sci. USA 86:2863-2867 (1989)) and because endothelin-like sequences are present in the COOH portions of the two known preproendothelins, it is probable that the exon duplication event preceded the gene duplication event.

Conservation of amino acids in the endothelin-like sequences as the endothelin genes diverged after duplication suggests an important physiologic function for these sequences. Examples of peptide hormone precursors containing more than one biologically active product include those for adrenocorticotrophic hormone (Nakanishi, S., et al., Nature 278:423-427 (1979)) and enkephalin (Nods, M., et al., Nature 295:202-206 (1982)).

Chromosomal Assignment of the ET3 Gene. To determine whether the genes encoding ET1 and ET3 are genetically linked, DNA isolated from human-mouse somatic hybrid cell lines was used to assign the endothelin genes to human chromosomes. HindIII-digested DNAs from hybrid and parental cell lines were examined by Southern blot analysis for the presence or absence of the human ET3 gene. The 0.5-kb EcoRI-BglII ET3 genomic fragment hybridized to human and murine HindIII restriction fragments of approximately 10 and 13 kb, respectively. The presence of human sequences hybridizing to the ET3 genomic probe correlated with the presence of human chromosome 20 (Table 2). We previously showed that the ET1 gene localized to human chromosome 6 (Bloch, K.D., et al., J. Biol. Chem. 264:10851-10857 (1989)); therefore, the genes encoding endothelin-related peptides are not clustered and are, in fact, located on distinct human chromosomes. (The chromosomal loci of the genes encoding ET1 and ET3 have been designated EDN1 and EDN3, respectively.

Expression of ET1 and ET3 Genes. To investigate the expression of the ET3 gene, RNA from human cells and tissues was subjected to RNA blot hybridization analysis with a restriction fragment derived from the 3′-untranslated region of the ET3 cDNA. This probe did not hybridize with ET1 mRNA. Abundant ET3 mRNA was found in human fetal lung, but ET3 mRNA was not detected in human umbilical vein endothelial cells that had been maintained in culture for four passages (Fig. 5A). The ET3 gene was also expressed in fetal spleen and pancreas, and a smaller amount of ET3 mRNA was observed in kidney (Fig. 5B). The isolation of two cDNA clones from a human hypothalamic cDNA library suggests that the ET3 gene is expressed, at least at low levels, in human hypothalamus. Yoshizawa et al. (Yoshizawa, T., et al., J. Cardiovasc. Pharmacol. 13:Suppl. 5, S216-S217 (1989)) observed that ET1 produces ventral root depolarization in newborn rat spinal cords. If, like ET1, ET3 modulates neuronal function, the presence of ET3 mRNA in the hypothalamus may suggest a role for ET3 in neurotransmission.

To compare ET3 and ET1 gene expression, a restriction fragment derived from the 3′-untranslated region of the ET1 cDNA was used. ET1 mRNA was observed in human fetal lung, spleen, and pancreas, and low levels of mRNA were present in fetal atrium and ventricle (Fig. 5B). In contrast to ET3 mRNA, abundant ET1 mRNA was found in human umbilical vein endothelial cells in culture (passage 4; data not shown). Except for ET1 mRNA in human umbilical vein endothelial cells, Inoue et al. (Inoue, A., et al., Proc. Natl. Acad. Sci. USA 86:2863-2867 (1989)) were unable to detect expression of the endothelin genes in human tissues. This apparent discrepancy between our observations and those of Inoue et al., (Inoue, A., et al., Proc. Natl. Acad. Sci. USA 86:2863-2867 (1989)) may be attributable to developemental modulation of endothelin gene expression.

The ET1 and ET3 genes are both expressed in fetal lung, spleen, and pancreas. The genes may be coexpressed in one or more cell types within these tissues. Expression of the two endothelin genes is discordant in endothelial cells and, probably, in fetal cardiac and renal tissues. A comparison of the 5′-flanking regions of th ET1 and ET3 genes should provide insight into the regulatory sequences important for coordinate and independent expression of the two genes.

Koseki et al. (Koseki, C., et al., Am. J. Physiol. 256:R858-R866 (1989)) observed binding sites for $^{125}$I-labeled ET1 in multiple rat tissues including arteries, myocardium, brain, kidney, lung, adrenal gland, intestine, and spleen. (The specificities of these binding sites for ET1, ET2 and ET3 were not determined.) Expression of ET1 and ET3 genes in some of the tissues containing ET1 binding sites suggests paracrine functions for ET1 and ET3. The presence of binding sites in tissues in which neither the ET1 nor the ET3 gene is expressed raises the possibility that other endothelin-related genes are expressed in these tissues, or that one or more of the endothelins is a circulating hormone.

Characterization of the mRNA encoding ET3 provides further evidence at the genetic level for a family of peptides which may participate in the regulation of vascular tone. In addition, if ET3 shares with ET1 the ability to induce mitogenesis, ET1 and ET3 may be important for the growth and development of the tissues in which they are expressed. The existence of two or more independently regulated genes encoding endothelin-related peptides implies that the physiologic response to local changes in blood flow or to changes in systemic blood pressure may be more complex than previously suspected. Investigations of this newly described family of genes may improve our understanding of the mechanisms involved in cardiovascular disorders such as hypertension and coronary artery vasospasm.

Modifications of the above-described modes for carrying out the invention that may be known to persons of skill in medicine, immunology, hybridoma technology, pharmacology, and/or related fields are intended to be within the scope of the following claims.

All publications and patent applications mentioned in this specification are indicative of the level of skill of those skilled in the art to which this invention pertains. All publications and patent applications are herein incorporated by reference to the same extent as if each individual publication or patent application was specifically and individually indicated to be incorporated by reference.

# Table 2

Segregation of a DNA probe for the ET3 gene with human chromosomes in HindIII-digested human-mouse cell

| Hybrid | Endo-thelin 3 | Human Chromosomes | | | | | | | | | | | | | | | | | | | | | | | Transloca-tions |
|---|---|---|---|---|---|---|---|---|---|---|---|---|---|---|---|---|---|---|---|---|---|---|---|---|---|
| | | 1 | 2 | 3 | 4 | 5 | 6 | 7 | 8 | 9 | 10 | 11 | 12 | 13 | 14 | 15 | 16 | 17 | 18 | 19 | 20 | 21 | 22 | X | |
| TSL-2 | + | - | + | t | - | + | + | - | - | - | + | - | + | - | - | - | - | t | + | - | + | + | - | + | 17/3,3/17 |
| XOL-6 | + | t | - | - | - | + | + | + | - | - | + | - | + | - | + | - | - | + | - | + | + | - | + | t | 1/X |
| WIL-15 | + | - | + | + | + | - | + | + | - | - | + | + | + | + | + | + | - | + | + | - | + | + | - | + | |
| WIL-6 | + | - | + | - | + | + | + | + | + | - | + | + | - | - | + | - | - | + | - | + | + | + | - | + | |
| XTR-2 | + | - | - | t | - | + | - | - | + | - | + | - | + | + | + | - | - | - | + | - | + | + | - | t | 3/X |
| XER-11 | + | + | - | + | + | + | + | + | + | + | + | + | t | + | - | - | + | + | + | + | + | + | + | t | 11/X,X/11 |
| NSL-9 | + | - | - | - | - | + | - | - | + | t | + | - | + | + | + | + | + | + | - | - | + | + | + | - | 17/9 |
| VTL-6 | + | - | + | - | - | - | + | + | + | - | + | + | - | - | - | = | - | + | - | + | + | + | + | - | |
| XOL-13 | + | t | - | - | + | + | + | - | + | - | - | + | + | + | - | - | - | - | + | - | - | + | - | + | X/1 |
| WIL-8X | + | - | - | + | + | + | + | - | + | + | - | + | + | + | - | + | - | - | + | + | + | + | + | - | + |
| DUM-13 | + | + | + | + | - | + | + | + | - | - | + | + | + | - | + | t | + | + | + | + | + | + | + | t | X/15,15/X |
| WIL-8Y | + | - | - | + | - | + | - | + | + | - | + | + | - | - | + | + | + | + | + | + | + | + | + | t | |
| XTR-3BSAgH | + | + | - | + | + | + | - | - | - | + | + | - | + | - | + | - | - | - | - | - | + | + | - | t | 3/X,10q- |
| NSL-16 | + | - | - | + | + | + | - | + | + | t | + | - | + | - | + | + | + | + | - | + | + | + | - | - | 17/9 |
| JSR-17S | + | + | + | + | - | + | - | t | + | + | + | + | + | + | + | + | + | + | - | + | + | + | - | | 7/9 |
| JSR-14 | + | - | + | + | + | + | + | - | - | - | - | - | + | + | - | - | - | + | - | - | + | + | - | + | |
| TSL-1 | + | - | + | + | + | + | - | - | - | - | + | + | + | - | + | - | + | + | + | - | + | + | - | | |
| VTL-8 | + | - | - | - | - | - | - | - | - | - | + | - | + | - | + | - | + | - | + | + | + | - | | |
| REW-7 | + | + | + | + | + | + | + | + | + | - | + | + | + | + | + | + | - | + | + | + | + | + | + | + | |
| VTL-7 | + | - | - | - | - | - | - | t | - | - | + | - | - | + | - | + | - | + | + | + | + | + | - | 7q- |
| WIL-5 | - | - | - | - | + | - | - | + | - | + | - | + | - | - | + | - | - | + | - | - | + | - | + | |
| SIR-11 | - | - | - | - | - | - | - | + | - | - | + | - | - | + | - | - | - | - | - | + | + | + | |
| REX-11BSHF | - | - | - | + | - | - | - | - | - | - | + | - | - | + | - | - | - | + | - | - | - | t | t | 22/X |
| SIR-8 | - | + | + | + | + | + | - | + | + | + | + | + | + | + | + | + | + | + | + | - | + | + | + | |
| WIL-14 | - | - | - | + | - | + | + | - | + | + | - | + | - | - | + | + | - | + | - | - | - | + | |
| DUA-β | - | - | + | - | + | + | - | - | - | - | + | - | + | - | + | + | - | - | + | |
| REX-11BSagB | - | - | - | + | - | - | - | - | - | - | + | - | - | + | + | - | - | + | - | - | - | - | |
| REX-57BSHB | - | - | - | - | - | - | - | - | - | - | - | + | - | - | + | - | - | + | - | - | - | + | t | t | 22/X |
| XOL-9 | - | t | + | + | + | - | + | - | - | - | + | - | + | - | + | - | + | + | + | - | + | + | + | X/1 |
| WIL-7 | - | + | + | + | - | + | + | - | + | - | + | - | + | + | - | + | + | - | + | - | + | |
| WIL-1 | - | - | - | - | - | + | - | - | + | - | + | - | + | + | - | + | - | + | - | + | |
| JSR-2 | - | - | + | + | - | - | + | - | - | - | + | - | + | - | - | - | - | + | + | |
| ATR-13 | - | + | + | + | + | + | + | + | + | + | - | + | + | + | + | + | + | + | + | - | - | + | t | 5/X |
| DUA-2BSAGA | - | - | + | - | - | - | - | + | + | - | - | - | + | + | - | - | + | - | - | - | - | - | |

EP 0 484 017 A2

Chromosomes

| Hybrids | 1 | 2 | 3 | 4 | 5 | 6 | 7 | 8 | 9 | 10 | 11 | 12 | 13 | 14 | 15 | 16 | 17 | 18 | 19 | 20 | 21 | 22 | X |
|---|---|---|---|---|---|---|---|---|---|---|---|---|---|---|---|---|---|---|---|---|---|---|---|
| No. of concordant | 16 | 17 | 17 | 18 | 24 | 21 | 19 | 17 | 15 | 24 | 24 | 24 | 16 | 16 | 17 | 19 | 22 | 16 | 19 | 34 | 25 | 18 | 18 |
| No. of discordant | 15 | 17 | 15 | 16 | 10 | 13 | 13 | 17 | 17 | 10 | 9 | 10 | 18 | 18 | 16 | 15 | 11 | 18 | 15 | 0 | 9 | 14 | 16 |
| Percent discordance | 48 | 50 | 47 | 47 | 29 | 38 | 41 | 50 | 53 | 29 | 27 | 29 | 53 | 53 | 48 | 44 | 33 | 53 | 44 | 0 | 26 | 44 | 62 |

Southern blots were prepared from HindIII-digested DNA from 34 human-mouse somatic hybrid cell lines and their parental cells. Blots were hybridized with a $^{32}$P-labeled EcoRI-BglII restriction fragment of the ET3 gene. Scoring for the human ET3 gene was determined by the presence (+) or the absence (-) of a human 10-kb HindIII restriction fragment that hybridized with the radiolabeled ET3 gene probe. Concordant hybrids have either retained or lost the human restriction fragment together with a specific human chromosome. Discordant hybrids have either retained the human restriction fragment but not a specific chromosome, or the converse. Percent discordance indicates the degree of discordant segregation for a marker and a chromosome. A 0% discordance is the basis for chromosome assignment. The ET3 gene is assigned to human chromosome 20. (Chromosomal translocations are indicated by "t," and the specific translocation is indicated to the right.)

SEQUENCE LISTING

1.  SEQ ID NO:1:

    (i) SEQUENCE CHARACTERISTICS:
        (A) LENGTH: 28 base pairs
        (B) TYPE: nucleic acid
        (C) STRANDEDNESS: single
        (D) TOPOLOGY: linear

    (ii) MOLECULE TYPE: DNA


    GGGAATTCAA GGGCCAGGCT GCTGCCAC                          28


2.  SEQ ID NO:2:

    (i) SEQUENCE CHARACTERISTICS:
        (A) LENGTH: 28 base pairs
        (B) TYPE: nucleic acid
        (C) STRANDEDNESS: single
        (D) TOPOLOGY: linear

    (ii) MOLECULE TYPE: DNA


    GGGAATTCAG CTCCCCCTGC CCCCAATC                          28


3.  SEQ ID NO:3:

    (i) SEQUENCE CHARACTERISTICS:
        (A) LENGTH: 1261 base pairs
        (B) TYPE: nucleic acid
        (C) STRANDEDNESS: both
        (D) TOPOLOGY: linear

    (ii) MOLECULE TYPE: DNA (genomic)


CCAGCTTAAT AGCAGGACGC TGGCAACAGG CACTCCCTGC TCCAGTCCAG CCTGGCGCTC    60

CACCGCCGCT ATGGTCTCCG TGCCTACCAC CTGGTGCTCC GTTGCGCTAG CCCTGCTCGT   120

GGCCCTGCAT GAAGGGAAGG GCCAGGCTGC TGCCACCCTG GAGCAGCCAG CGTCCTCATC   180

TCATGCCCAA GGCACCCACC TTCGGCTTCG CCGTTGCTCC TGCAGCTCCT GGCTCGACAA   240

GGAGTGCGTC TACTTCTGCC ACTTGGACAT CATCTGGGTG AACACTCCTG AACAGACAGC   300

TCCTTACGGC CTGGGAAACC CGCCAAGACG CCGGCGCCGC TCCCTGCCAA GGCGCTGTCA   360

GTGCTCCAGT GCCAGGGACC CCGCCTGTGC CACCTTCTGC CTTCGAAGGC CCTGGACTGA   420

AGCCGGGGCA GTCCCAAGCC GGAAGTCCCC TGCAGACGTG TTCCAGACTG GCAAGACAGG   480

```
GGCCACTACA GGAGAGCTTC TCCAAAGGCT GAGGGACATT TCCACAGTCA AGAGCCTCTT      540

TGCCAAGCGA CAACAGGAGG CCATGCGGGA GCCTCGGTCC ACACATTCCA GGTGGAGGAA      600

GAGATAGTGT CGTGAGCTGG AGGAACATTG GGAAGGAAGC CCGCGGGGAG AGAGGAGGAG      660

AGAAGTGGCC AGGGCTTGTG GACTCTCTGC CTGCTTCCTG GACCGGGGCC TTGGTCCCAG      720

ACAGCTGGAC CCATTTGCCA GGATTGGCAC AAGCTCCCTG GTGAGGGAGC CTCGTCCAAG      780

GCAGTTCTGT GTCCTCGCAC TGCCCAGGGA AGCCCTCGGC CTCCAGACTG CGGAGCAGCC      840

TCCAGTGCTG GCTGCTGGCC CACAGCTCTG CTGGAAGAAC TGCATGGGGA GTACATTCAT      900

CTGGAGGCTG CGTCCTGAGG AGTGTCCTGT CTGCTGGGCT ACAAACCAGG AGCAACCGTG      960

CAGCCACGAA CACGCATGCC TCAGCCAGCC CTGGAGACTG GATGGCTCCC CTGAGGCTGG     1020

CATCCTGGCT GGCTGTGTCC TCTCCAGCTT TCCCTCCCCA GAGTTCTTGC ACCCTCATTC     1080

CCTCGGGACC CTCCCAGTGA GAAGGGCCTG CTCTGCTTTT CCTGTCTGTA TATAACTTAT     1140

TTGCCCTAAG AACTTTGAGA ATCCCAATTA TTTATTTTAA TGTATTTTTT AGACCCTCTA     1200

TTTACCTGCG AACTTGTGTT TATAATAAAT GAGGAAACAT AAAAAAAAAA AAAAAAAAAA     1260

A                                                                     1261
```

4.   SEQ ID NO:4:

    (i) SEQUENCE CHARACTERISTICS:
       (A) LENGTH: 178 amino acids
       (B) TYPE: amino acid
       (D) TOPOLOGY: linear

    (ii) MOLECULE TYPE: protein

```
Met Val Ser Val Pro Thr Thr Trp Cys Ser Val Ala Leu Ala Leu Leu
1               5                   10                  15

Val Ala Leu His Glu Gly Lys Gly Gln Ala Ala Ala Thr Leu Glu Gln
            20                  25                  30

Pro Ala Ser Ser Ser His Ala Gln Gly Thr His Leu Arg Leu Arg Arg
            35                  40                  45

Cys Ser Cys Ser Ser Trp Leu Asp Lys Glu Cys Val Tyr Phe Cys His
    50                  55                  60

Leu Asp Ile Ile Trp Val Asn Thr Pro Glu Gln Thr Ala Pro Tyr Gly
65                  70                  75                  80

Leu Gly Asn Pro Pro Arg Arg Arg Arg Arg Ser Leu Pro Arg Arg Cys
                85                  90                  95
```

```
Gln Cys Ser Ser Ala Arg Asp Pro Ala Cys Ala Thr Phe Cys Leu Arg
        100                     105                 110

Arg Pro Trp Thr Glu Ala Gly Ala Val Pro Ser Arg Lys Ser Pro Ala
        115                     120                 125

Asp Val Phe Gln Thr Gly Lys Thr Gly Ala Thr Thr Gly Glu Leu Leu
    130                     135                 140

Gln Arg Leu Arg Asp Ile Ser Thr Val Lys Ser Leu Phe Ala Lys Arg
145                     150                 155                 160

Gln Gln Glu Ala Met Arg Glu Pro Arg Ser Thr His Ser Arg Trp Arg
                165                 170                 175

Lys Arg
```

5. SEQ ID NO:5:

   (i) SEQUENCE CHARACTERISTICS:
       (A) LENGTH: 2233 base pairs
       (B) TYPE: nucleic acid
       (C) STRANDEDNESS: both
       (D) TOPOLOGY: linear

   (ii) MOLECULE TYPE: DNA (genomic)

```
CGGGTAGCGC GCTCTGAAAG TTTATGACCG CCGCAGCCAA CTCCTGGCCG GAGCTGGAGA    60

CGCAGCGAGC GATCGGCCGG CCTCGAACCC CCACAGCTGG AGGGCGAGGC CAGCTGTACC   120

CGGCCCCAGT GCCCTTTCGC GGCCACAAGC GGCCGTCCTC CTGGTCCGGT GCTCCGGCGC   180

CTGATCTAGG TTCATGGAGC CGGGGCTGTG GCTCCTTTTC GGGCTCACAG TGACCTCCGC   240

CGCAGGATTC GTGCCTTGCT CCCAGTCTGG GGATGCTGGC AGGCGCGGCG TGTCCCAGGC   300

CCCCACTGCA GCCAGATCTG AGGGGGACTG TGAAGAGACT GTGGCTGGCC CTGGCGAGGA   360

GACTGTGGCT GGCCCTGGCG AGGGGACTGT GGCCCCGACA GCACTGCAGG GTCCAAGCCC   420

TGGAAGCCCT GGGCAGGAGC AGGCGGCCGA GGGGGCCCCT GAGCACCACC GATCCAGGCG   480

CTGCACGTGC TTCACCTACA AGGACAAGGA GTGTGTCTAC TATTGCCACC TGGACATCAT   540

TTGGATCAAC ACTCCCGAAC AGACGGTGCC CTATGGACTG TCCAACTACA GAGGAAGCTT   600

CCGGGGCAAG AGGTCTGCGG GGCCACTTCC AGGGAATCTG CAGCTCTCAC ATCGGCCACA   660

CTTGCGCTGC GCTTGTGTGG GGAGATATGA CAAGGCCTGC CTGCACTTTT GCACCCAAAC   720

TCTGGACGTC AGCAGTAATT CAAGGACGGC AGAAAAAACA GACAAAGAAG AGGAAGGGAA   780

GGTTGAAGTC AAGGACCAAC AAAGCAAGCA GGCTTTAGAC CTCCACCATC CAAAGCTCAT   840
```

```
GCCCGGCAGT GGACTCGCCC TCGCTCCATC TACCTGCCCC CGCTGCCTCT TTCAGGAAGG      900
AGCCCCTTAG GAGGACAGGC CTGCAGCTCC AATTTCATGC AGGAAATTGG TTTTGGAGAG      960
TTTTGGCAAG TTGGAAAGCC ACTTACTGGC TTTTGACATG ACTTCTCTTG GAGAATAAGT     1020
GGACTCCAAG CTAACTCTTT GCAAATGTAA ACACATGTCC ATCTTGTTAA TAAATGCAAA     1080
ATGCCCGTGC AGCAGAAGCA TGCGACTTTC ATATCCTTGC CTAGAATAGG CTGCATGGTG     1140
TATGTCAGTG AGGGCCACGA GGCGTCGGCT TTAGACACAG ATCATAGCTC TACAGGAGTT     1200
TATGAATTTG AAGCTTATGG GATTTTGGCA GAGAAATTTT CAGCTGTGCT TGATACCCAC     1260
CAAAAGAATG TATCTCGAAA GAATGAAGGA AGAAGAAAAA AGGATCCTTG ATGTTTGTGA     1320
CAAGAAAATG AGAAAGTTAG TATCTGCAAT ACAGAGCTTG TTCCTGTTCA GTGACTGACC     1380
CTCTGTATTC TGTATAGACA CCAGGCCGAT ACACAGTGGA GTTCCCAGGC CTTGTTTGCA     1440
GGAAGCCGAC TGTAAAGACA GCCCCAGCTC AAGGCTATTA GGTTGAATAT TTGCTTTCAT     1500
GAGTAAATGT GGATCTTTGG GGAATGGCTT CAAAATAAGT CACGAACACA AATTCTTTGT     1560
AAATTATGTA AATTCCTGTT TATATAAATT GGCAACAACT TATACCGTCT GACAGTTCAA     1620
AATCTCTTTC AGCTGCGCTC TTCCCACCGA GCCGAGCTTA CTGTGAGTGT GGAGATGTTA     1680
TCCCACCATG TAAAGTCGCC TGCGCAGGGG AGGGCTGCCC ATCTCCCCAA CCCAGTCACA     1740
GAGAGATAGG AAACGGCATT TGAGTGGGTG TCCAGGGCCC CGTAGAGAGA CATTTAAGAT     1800
GGTGTATGAC AGAGCATTGG CCTTGACCAA ATGTTAAATC CTCTGTGTGT ATTTCATAAG     1860
TTATTACAGG TATAAAAGTG ATGACCTATC ATGAGGAAAT GAAAGTGGCT GATTTGCTGG     1920
TAGGATTTTG TACAGTTTAG AGAAGCGATT ATTTATTGTG AAACTGTTCT CCACTCCAAC     1980
TCCTTTATGT GGATCTGTTC AAAGTAGTCA CTGTATATAC GTATAGAGAG GTAGATAGGT     2040
AGGTAGATTT TAAATTGCAT TCTGAATACA AACTCATACT CCTTAGAGCT TGAATTACAT     2100
TTTTAAAATG CATATGTGCT GTTTGGCACC GTGGCAAGAT GGTATCAGAG AGAAACCCAT     2160
CAATTGCTCA AATACTCAGA AAGTACTGTC AAAAGCCTAA TAAAAAACCT AAAGTTTGCT     2220
CTGAAAAAAA AAA                                                        2233
```

6.  SEQ ID NO:6:

    (i) SEQUENCE CHARACTERISTICS:
        (A) LENGTH: 238 amino acids
        (B) TYPE: amino acid
        (D) TOPOLOGY: linear

    (ii) MOLECULE TYPE: protein


```
Met Glu Pro Gly Leu Trp Leu Leu Phe Gly Leu Thr Val Thr Ser Ala
1               5                   10              15

Ala Gly Phe Val Pro Cys Ser Gln Ser Gly Asp Ala Gly Arg Arg Gly
            20              25              30

Val Ser Gln Ala Pro Thr Ala Ala Arg Ser Glu Gly Asp Cys Glu Glu
        35              40              45

Thr Val Ala Gly Pro Gly Glu Glu Thr Val Ala Gly Pro Gly Glu Gly
        50              55              60

Thr Val Ala Pro Thr Ala Leu Gln Gly Pro Ser Pro Gly Ser Pro Gly
65              70              75              80

Gln Glu Gln Ala Ala Glu Gly Ala Pro Glu His His Arg Ser Arg Arg
                85              90              95

Cys Thr Cys Phe Thr Tyr Lys Asp Lys Glu Cys Val Tyr Tyr Cys His
            100             105             110

Leu Asp Ile Ile Trp Ile Asn Thr Pro Glu Gln Thr Val Pro Tyr Gly
            115             120             125

Leu Ser Asn Tyr Arg Gly Ser Phe Arg Gly Lys Arg Ser Ala Gly Pro
    130             135             140

Leu Pro Gly Asn Leu Gln Leu Ser His Arg Pro His Leu Arg Cys Ala
145             150             155             160

Cys Val Gly Arg Tyr Asp Lys Ala Cys Leu His Phe Cys Thr Gln Thr
            165             170             175

Leu Asp Val Ser Ser Asn Ser Arg Thr Ala Glu Lys Thr Asp Lys Glu
        180             185             190

Glu Glu Gly Lys Val Glu Val Lys Asp Gln Gln Ser Lys Gln Ala Leu
        195             200             205

Asp Leu His His Pro Lys Leu Met Pro Gly Ser Gly Leu Ala Leu Ala
    210             215             220

Pro Ser Thr Cys Pro Arg Cys Leu Phe Gln Glu Gly Ala Pro
225             230             235
```

**Claims**

1. A peptide which is capable of being cleaved to form ET-2 or a variant thereof.

2. A peptide according to claim 1 which comprises the amino acid sequence of Figure 2 and/or is encoded by the nucleotide sequence given in Figure 2.

3. A peptide which is capable of being cleaved to form endothelin-3 or a variant thereof.

4. A peptide according to claim 3 which comprises the amino acid sequence of Figure 3 and/or is encoded by the nucleotide sequence given in Figure 3.

5. A peptide which is capable of being cleaved to form ET-2 and/or ET-3, or a variant thereof, for use in medicine.

6. A pharmaceutical composition comprising a peptide which is capable of being cleaved to form ET-2 and/or ET-3, or a variant thereof, and a pharmaceutically acceptable carrier.

7. The use of a peptide which is capable of being cleaved to form ET-2 and/or ET-3, or a variant thereof, in the preparation of:
    (a) a blood pressure and/or vascular activity regulating or modulating agent;
    (b) a vasoconstrictor and/or pressor agent;
    (c) a regional vasodilatory agent;
    (d) a vascular smooth muscle cell or fibroblast stimulating or proliferating agent;
    (e) an airway and/or intestinal smooth muscle contracting agent;
    (f) a myocardium positive inotropic and/or chronotropic agent;
    (g) an iconsanoid or vascular bed endothelium-derived relaxing factor releasing agent;
    (h) an atrial natriuretic peptide from atrial cardiocyte stimulating agent;
    (i) a renin release from glomerulus inhibiting agent; and/or
    (j) a norepinephrine release from sympathetic terminal modulating agent.

8. A product comprising a peptide which is capable of being cleaved to form ET-2 and/or ET-3, or a variant thereof, and a cleavage agent adapted to cleave a peptide to form ET-2 and/or ET-3, or a variant thereof, as a combined preparation for simultaneous, separate or sequential use in vascular, cardiovascular, blood pressure regulatory, vasoconstrictor, vasodilatory and/or pressor therapy.

9. A product as claimed in claim 8 wherein the cleavage agent is a proteolytic enzyme.

10. A receptor molecule capable of binding an endothelin which is ET-1, ET-2 and/or ET-3, or a functional derivative thereof.

**Claims for the following Contracting States: ES, GR**

1. A process for the preparation of a peptide which is capable of being cleaved to form ET-2 or a variant thereof, the process comprising coupling successive amino acid residues and/or coupling amino acid sequences together.

2. A process according to claim 1 wherein the peptide comprises the amino acid sequence of Figure 2 and/or is encoded by the nucleotide sequence given in Figure 2.

3. A process for the preparation of a peptide which is capable of being cleaved to form endothelin-3 or a variant thereof.

4. A process according to claim 3 wherein the peptide comprises the amino acid sequence of Figure 3 and/or is encoded by the nucleotide sequence given in Figure 3.

5. A process for the preparation of a pharmaceutical composition, the process comprising admixing a peptide which is capable of being cleaved to form ET-2 and/or ET-3, or a variant thereof, and a pharmaceutically acceptable carrier.

6. The use of a peptide which is capable of being cleaved to form ET-2 and/or ET-3, or a variant thereof, in the preparation of:
(a) a blood pressure and/or vascular activity regulating or modulating agent;
(b) a vasoconstrictor and/or pressor agent;
(c) a regional vasodilatory agent;
(d) a vascular smooth muscle cell or fibroblast stimulating or proliferating agent;
(e) an airway and/or intestinal smooth muscle contracting agent;
(f) a myocardium positive inotropic and/or chronotropic agent;
(g) an iconsanoid or vascular bed endothelium-derived relaxing factor releasing agent;
(h) an atrial natriuretic peptide from atrial cardiocyte stimulating agent;
(i) a renin release from glomerulus inhibiting agent; and/or
(j) a norepinephrine release from sympathetic terminal modulating agent.

7. A process for the preparation of a receptor molecule capable of binding an endothelin which is ET-1, ET-2 and/or ET-3, or a functional derivative thereof the process comprising coupling successive amino acid residues and/or coupling amino acid sequences together.

HTB 119

28 S −

18 S −

FIGURE 1

```
ccagcttaataagcaggacgctggcaacaggcactccctgctccagtccagcctggcgctccaccgccgct            (70)

atg gtc tcc gtg cct acc acc tgg tgc tcc gtt gcg cta gcc ctg ctc gtg gcc ctg cat
MET VAL SER VAL PRO THR THR TRP CYS SER VAL ALA LEU ALA LEU LEU VAL ALA LEU HIS      20

gaa ggg aag ggc cag gct gct gcc acc ctg gag cag cca gcg tcc tca tct cat gcc caa
GLU GLY LYS GLY GLN ALA Ala Ala Thr Leu Glu Gln Pro Ala Ser Ser Ser His Ala Gln      40

ggc acc cac ctt cgg ctt cgc cgt tgc tcc tgc agc tcc tgg ctc gac aag gag tgc gtc
Gly Thr His Leu Arg Leu Arg Arg Cys Ser Cys Ser Ser Trp Leu Asp Lys Glu Cys Val      60

tac ttc tgc cac ttg gac atc atc tgg gtg aac act cct gaa cag aca gct cct tac ggc
Tyr Phe Cys His Leu Asp Ile Ile Trp Val Asn Thr Pro Glu Gln Thr Ala Pro Tyr Gly      80

ctg gga aac ccg cca aga cgc cgg cgc cgc tcc ctg cca agg cgc tgt cag tgc tcc agt
Leu Gly Asn Pro Pro Arg Arg Arg Arg Arg Ser Leu Pro Arg Arg Cys Gln Cys Ser Ser      100

gcc agg gac ccc gcc tgt gcc acc ttc tgc ctt cga agg ccc tgg act gaa gcc ggg gca
Ala Arg Asp Pro Ala Cys Ala Thr Phe Cys Leu Arg Arg Pro Trp Thr Glu Ala Gly Ala      120

gtc cca agc cgg aag tcc cct gca gac gtg ttc cag act ggc aag aca ggg gcc act aca
Val Pro Ser Arg Lys Ser Pro Ala Asp Val Phe Gln Thr Gly Lys Thr Gly Ala Thr Thr      140

gga gag ctt ctc caa agg ctg agg gac att tcc aca gtc aag agc ctc ttt gcc aag cga
Gly Glu Leu Leu Gln Arg Leu Arg Asp Ile Ser Thr Val Lys Ser Leu Phe Ala Lys Arg      160

caa cag gag gcc atg cgg gag cct cgg tcc aca cat tcc agg tgg agg aag aga            (604)
Gln Gln Glu Ala Met Arg Glu Pro Arg Ser Thr His Ser Arg Trp Arg Lys Arg            178
                                                                   [SEQ ID NO:4]

tagtgtcgtgagctggaggaacattgggaaggaagcccgcggggagagaggaggagagaagtggccagggcttgtggac
tctctgcctgcttcctggaccggggccttggtcccagacagctggacccatttgccaggattggcacaagctccctggt
gagggagcctcgtccaaggcagttctgtgtcctcgcactgcccagggaagccctcggcctccagactgcggagcagcct
ccagtgctggctgctggcccacagctctgctggaagaactgcatggggagtacattcatctggaggctgcgtcctgagg
agtgtcctgtctcgctgggctacaaaccaggagcaaccgtgcagccacgaacacgcatgcctcagccagccctggagact
ggatggctcccctgaggctggcatcctggctggctgtgtcctctccagctttccctccccagagttcttgcaccctcat
tccctcgggaccctccagtgagaaggggcctgctctgcttttcctgtctgtatataacttatttgccctaagaactttg
agaatcccaattatttattttaatgtattttttagaccctctatttacctgcgaacttgtgtttataataaatgaggaa
acataaaaaaaaaaaaaaaaaaaaaaa [SEQ ID NO:3]                                      (1261)
```

FIGURE 2

cgggtagcgcgctctgaaagtttatgaccgccgcagccaactcctggccggagctggagacgcagcgagcgatcggccg
gcctcgaaccccacagctggagggcgaggccagctgtacccggccccagtgccctttcgcggccacaagcggccgtcc
tcctggtccggtgctccggcgcctgatctaggttc                                          (193)

atg gag ccg ggg ctg tgg ctc ctt ttc ggg ctc aca gtg acc tcc gcc gca gga ttc gtg
MET GLU PRO GLY LEU TRP LEU LEU PHE GLY LEU THR VAL THR SER ALA ALA GLY PHE VAL    20

cct tgc tcc cag tct ggg gat gct ggc agg cgc ggc gtg tcc cag gcc ccc act gca gcc
PRO CYS SER GLN SER Gly Asp Ala Gly Arg Arg Gly Val Ser Gln Ala Pro Thr Ala Ala    40
                    †

aga tct gag ggg gac tgt gaa gag act gtg gct ggc cct ggc gag gag act gtg gct ggc
Arg Ser Glu Gly Asp Cys Glu Glu Thr Val Ala Gly Pro Gly Glu Glu Thr Val Ala Gly    60

cct ggc gag ggg act gtg gcc ccg aca gca ctg cag ggt cca agc cct gga agc cct ggg
Pro Gly Glu Gly Thr Val Ala Pro Thr Ala Leu Gln Gly Pro Ser Pro Gly Ser Pro Gly    80

cag gag cag gcg gcc gag ggg gcc cct gag cac cac cga tcc agg cgc tgc acg tgc ttc
Gln Glu Gln Ala Ala Glu Gly Ala Pro Glu His His Arg Ser Arg Arg Cys Thr Cys Phe    100

acc tac aag gac aag gag tgt gtc tac tat tgc cac ctg gac atc att tgg atc aac act
Thr Tyr Lys Asp Lys Glu Cys Val Tyr Tyr Cys His Leu Asp Ile Ile Trp Ile Asn Thr    120

ccc gaa cag acg gtg ccc tat gga ctg tcc aac tac aga gga agc ttc cgg ggc aag agg
Pro Glu Gln Thr Val Pro Tyr Gly Leu Ser Asn Tyr Arg Gly Ser Phe Arg Gly Lys Arg    140

tct gcg ggg cca ctt cca ggg aat ctg cag ctc tca cat cgg cca cac ttg cgc tgc gct
Ser Ala Gly Pro Leu Pro Gly Asn Leu Gln Leu Ser His Arg Pro His Leu Arg Cys Ala    160

tgt gtg ggg aga tat gac aag gcc tgc ctg cac ttt tgc acc caa act ctg gac gtc agc
Cys Val Gly Arg Tyr Asp Lys Ala Cys Leu His Phe Cys Thr Gln Thr Leu Asp Val Ser    180

agt aat tca agg acg gca gaa aaa aca gac aaa gaa gag gaa ggg aag gtt gaa gtc aag
Ser Asn Ser Arg Thr Ala Glu Lys Thr Asp Lys Glu Glu Glu Gly Lys Val Glu Val Lys    200

gac caa caa agc aag cag gct tta gac ctc cac cat cca aag ctc atg ccc ggc agt gga
Asp Gln Gln Ser Lys Gln Ala Leu Asp Leu His His Pro Lys Leu Met Pro Gly Ser Gly    220

ctc gcc ctc gct cca tct acc tgc ccc cgc tgc ctc ttt cag gaa gga gcc cct tag   (910)
Leu Ala Leu Ala Pro Ser Thr Cys Pro Arg Cys Leu Phe Gln Glu Gly Ala Pro END    238
                                                                  [SEQ ID NO:6]

gaggacaggcctgcagctccaatttcatgcaggaaattggtttttggagagttttggcaagttggaaagccacttactgg
cttttgacatgacttctctcttggagaataagtggactccaagctaactctttgcaaatgtaaacacatgtccatcttgtt
aataaatgcaaaatgcccgtgcagcagaagcatgcgactttcatatccttgcctagaataggctgcatggtgtatgtca
gtgagggccacgaggcgtcggctttagacacagatcatagctctacaggagtttatgaatttgaagcttatgggatttt
ggcagagaaattttcagctgtgtgcttgatacccaccaaaagaatgtatctcgaaagaatgaaggaagaagaaaaaaggat
ccttgatgtttgtgacaagaaaatgagaaagttagtatctgcaatacagagcttgttcctgttcagtgactgaccctct
gtattctgtatagacaccaggccgatacacagtggagttcccaggccttgtttgcaggaagccgactgtaaagacagcc
ccagctcaaggctattaggttgaatatttgctttcatgagtaaatgtggatctttggggaatggcttcaaaataagtca
cgaacacaaattctttgtaaattatgtaaattcctgtttatataaattggcaacaacttataccgtctgacagttcaaa
atctctttcagctgcgctcttcccaccgagccgagcttactgtgagtgtggagatgttatcccaccatgtaaagtcgcc
tgcgcaggggagggctgcccatctccccaacccagtcacagagataggaaacggcatttgagtgggtgtccagggcc
ccgtagagagacattaagatggtgtatgacagagcattggccttgaccaaatgttaaatcctctgtgtgtatttcata
agttattacaggtataaaagtgatgacctatcatgaggaaatgaaagtggctgattgctggtaggattttgtacagtt
tagagaagcgattatttattgtgaaactgttctccactccaactcctttatgtggatctgttcaaagtagtcactgtat
atacgtatagagaggtagataggtaggtagatttttaaattgcattctgaatacaaactcatactccttagagcttgaat
tacattttttaaaaatgcatatgtgctgtttggcaccgtggcaagatggtatcagagagaaacccatcaattgctcaaata
ctcagaaagtactgtcaaaagcctaataaaaaacctaaagtttgctctgaaaaaaaaaa [SEQ ID NO:5]       (2233)

FIGURE 3

FIGURE 4

FIGURE 5